# EUROPEAN PATENT APPLICATION

(11) **EP 4 844 778 A2**
(43) Date of publication of application: **25.11.2026**
(21) Application number: 26180743.2
(22) Date of filing: 22.05.2026
(51) Int. Cl.: G16H 20/17, A61B 5/00, G16H 20/60, G16H 40/63

(54) **ADAPTING FOR UNACCOUNTED EVENTS THAT AFFECT MEDICAMENT DELIVERY PARAMETERS**

(30) Priority: 23.05.2025 US 202563811177 P
(71) Applicant: Insulet Corporation, Acton, MA 01720 (US)
(72) Inventor: LEE, Joon Bok, Acton, 01720 (US); WHITELEY, William, Knoxville, 37932 (US); O'CONNOR, Jason, Acton, 01720 (US)
(74) Representative: Peterreins Schley

(57) **Abstract**

Disclosed are examples of an automated medicament delivery (AMD) algorithm and system that enable a user to update the AMD algorithm with information erroneously omitted from being input to the AMD algorithm. Users of the AMD system may partake in unaccounted events not accounted for by the AMD algorithm because a user fails to enter the event into their controller device because they became distracted, embarrassed, procrastinated, or the like. Examples of unaccounted events include manual delivery of medicament outside of the AMD system (e.g., a meal bolus); consumption of a meal or snack outside of typical meal or snack times, or in excess of or less than a previously estimated amount; over delivery of medicament, and the like. Additionally, the disclosed subject matter describes processes and devices for utilizing unaccounted event information regarding the unaccounted events to modify medicament delivery and AMD algorithm operation.

## Description

### CROSS-REFERENCE TO RELATED APPLICATION

This application claims the benefit of and priority to the U.S. Provisional Patent Application No. 63/811,177, filed on May 23, 2025, the entire content of which is incorporated herein by reference in its entirety.

### BACKGROUND

Automated medicament delivery (AMD) systems typically incorporate past medicament delivery history to personalize its medicament delivery recommendations and adapt to the medicament requirements of each user. However, these adaptations are typically based on an automated record of the user's medicament deliveries as recorded by the AMD system, and may not take into account any activities (e.g., meals or exercise), medicament deliveries entered manually, or other interactions relevant to each user's glucose control that the user may execute outside of the AMD system and that may have occurred a number of hours or days in the past.

Some AMD systems typically enable users to indicate their meal ingestions as part of their insulin therapy. However, due to the reliance on user manual input of these meals, there is a significant likelihood of incorrect or missed inputs, which both hamper the glucose control outcomes of these users, as well as reduce the ability of AMD systems to adapt to the users' insulin needs. What is needed is a process to detect potentially missed meals for users, and enable users to indicate that they ingested these meals even if they had initially missed entering those meals.

Other AMD systems may modify their medicament delivery recommendations based on known previous medicament delivery histories. However, users may also deliver additional medicament at different times, such as delivering insulin beyond meal ingestions, such as via manual injections. In the example of insulin, when an AMD algorithm recommends insulin deliveries without knowledge of these additional manual insulin deliveries, the uninformed recommendation may lead to erroneous insulin delivery and increased risk of hyperglycemia or hypoglycemia for the user. What is needed are algorithms and methods to enable users to indicate additional insulin deliveries that may not be otherwise known or inferred by the AMD algorithm, and allow the AMD algorithm to modify its behaviors accordingly and ensure its recommended insulin delivery is up-to-date and effective.

In some cases, users of AMD systems augment their insulin therapy with additional manual insulin deliveries. In typical cases, the users may utilize insulin delivery through the AMD system pumps, and thus additional indications provided by the user to the AMD algorithm beyond the standard insulin delivery commands to the system are unnecessary. However, there may be cases where the user's manual insulin delivery was based on erroneous information. As it is impossible to remove insulin once delivered, an AMD system that is unaware of the user's understanding of an erroneously high manual insulin delivery may be delayed in its response to this erroneous delivery. What is needed is an AMD system that enables users to inform the AMD system following completion of manual insulin delivery, and for the AMD systems to effectively respond to those user interactions.

### SUMMARY

Disclosed is an example of a medicament delivery system including a wearable medicament delivery device: a data storage, a display (in particular a touchscreen display), and a processor. The wearable medicament delivery device may be operable to communicate with an external device and to expel medicament from a reservoir. The data storage maintains treatment program parameters during an adaptation cycle of the wearable medicament delivery device. The processor is coupled to the display and operable to communicate with the wearable medicament delivery device and execute a control application. The processor may be operable to: evaluate the treatment program parameters for the adaptation cycle; receive, via a user interface presented on the display, additional unaccounted event information regarding an unaccounted event during the adaptation cycle; revise treatment program parameters based on the unaccounted event information; and estimate medicament dosages to be expelled from the wearable medicament delivery device.

Disclosed is another example of a medicament delivery system including a wearable medicament delivery device, a data storage, and a processor. The wearable medicament delivery device may be operable to communicate with an external device and to expel medicament from a reservoir. The data storage is operable to maintain a user history related to the wearable medicament delivery device. The processor is coupled to the data storage and operable to communicate with the wearable medicament delivery device and execute a control application. The processor may be operable to: present in a user interface prompts for a user to enter unaccounted event information for an event occurred at least a set time period prior to presentation of the prompt; receive the unaccounted event information; evaluate the received unaccounted event information; and based on a result of the evaluation, determine whether to adjust one or more of: a bolus amount, an amount of insulin on board, or a maximum amount of insulin to deliver in a next operational cycle.

Disclosed is another exemplary medicament delivery system. The medicament delivery system includes a wearable medicament delivery device, a data storage, a display; and a processor. The wearable medicament delivery device may be operable to communicate with an external device and to expel medicament from a reservoir. The data storage may maintain treatment program parameters during an adaptation cycle of the wearable medicament delivery device. The processor may be coupled to the display and operable to communicate with the wearable medicament delivery device and execute a control application. The processor may be operable to, after start of an extended bolus to compensate for a meal, present a user interface on the display enabling entry of a revised carbohydrate amount for the meal to replace a previous estimate. The processor, in response to the entry of the revised carbohydrate amount for the meal, determine a remainder of the started extended bolus yet to be delivered; calculate an updated dosage for the started extended bolus; revise the remainder of the started extended bolus yet to be delivered based on the calculated updated dosage for the started extended bolus; and cause the revised remainder to be expelled by the wearable medicament delivery device.

Another medicament delivery system is disclosed. The medicament delivery system may include a processor and a memory. The memory is operable to store instructions. The processor may execute the stored instructions and be operable to enter a first amount of carbohydrate for a meal into a bolus calculator, wherein the first amount of carbohydrate is an initial estimate of the amount of carbohydrate in the meal; generate a bolus dosage based on the entered first amount of carbohydrate; enter a second amount of carbohydrate for the meal into the bolus calculator, wherein the second amount of carbohydrate is an updated estimate of the first amount of carbohydrate in the meal; evaluate the first amount of carbohydrate and the second amount of carbohydrate; and generate an adjusted bolus dosage based on the entered first amount of carbohydrate, the entered second amount of carbohydrate, and the generated bolus dosage.

Another medicament delivery system is disclosed. The medicament delivery system may include a processor and a memory. The memory is operable to store instructions. The processor may execute the stored instructions and be operable to monitor a plurality of glucose values of a user obtained over a period of time with respect to a threshold value, wherein the threshold value is a value generated based on the glucose values; generate a factor using the glucose values; determine whether the factor exceeds the threshold; generate a prompt inquiring if extra insulin has been delivered for presentation on a user interface in response to the factor not exceeding the threshold, wherein a prompt is not generated when the factor does exceed the threshold; receive an input in response to the prompt; and modify medicament delivery parameters based on the received input.

### BRIEF DESCRIPTION OF THE DRAWINGS

To easily identify the discussion of any particular element or act, the most significant digit or digits in a reference number refer to the figure number in which that element is first introduced.
FIG. 1 illustrates an exemplary user interface that enables users to enter unaccounted event information according to embodiments of the disclosed subject matter.
FIG. 2 illustrates an exemplary process for obtaining unaccounted event information according to embodiments of the disclosed subject matter.
FIG. 3 illustrates another exemplary process for enabling users to provide feedback related to erroneous manual insulin deliveries according to the disclosed subject matter.
FIG. 4 illustrates an exemplary user interface suitable for implementing the process of FIG. 3.
FIG. 5 illustrates an exemplary process for algorithmic responses to user entries of unknown insulin deliveries according to the disclosed subject matter.
FIG. 6 illustrates another exemplary process for algorithmic responses to user entries of unknown insulin deliveries according to the disclosed subject matter.
FIG. 7 illustrates an exemplary process that enables a user to indicate mistaken inputs related to carbohydrate ingestion according to the disclosed subject matter.
FIG. 8 illustrates a graph showing benefits of additional and/or on-time bolusing in response to a missed meal according to examples disclosed herein.
FIG. 9A illustrates an exemplary user interface for initiating a process to update an amount of carbohydrates for a meal according to the disclosed subject matter.
FIG. 9B illustrates an exemplary process for updating an automated medicament delivery algorithm with an amount of carbohydrates for a meal according to the disclosed subject matter.
FIG. 10A illustrates an exemplary user interface for an exemplary process for updating of exogenous insulin delivery according to the disclosed subject matter.
FIG. 10B illustrates an exemplary process for updating the exogenous insulin delivery using the exemplary user interface according to the disclosed subject matter of FIG. 10A.
FIG. 11 illustrates an exemplary medicament delivery system operable to implement the examples disclosed herein.

### DETAILED DESCRIPTION

The following examples are intended to address instances where a user either ingests a meal but misses informing the AMD algorithm of the meal, inputs extra medicament beyond what the AMD algorithm recommends, or engages in physical activity that is not anticipated by the AMD algorithm (or the user) and the AMD algorithm responds by taking actions (which can include taking no actions) in response increases or decreases in glucose measurement values that appear as a typical deviation or excursion of the blood glucose to the AMD algorithm. Hence, the AMD algorithm may take action (including no actions or stopping actions) to merely return the glucose to the target set point without attempting to compensate for the unaccounted carbohydrate, medicament, or physical activity. In other words, the AMD algorithm responds to a glucose excursion, and not ingestion of a meal, excess medicament, missed medicament, sudden physical activity, or the like.

The following examples describe beneficial AMD systems and AMD algorithms and a process by which users could complete or update their past user history to include events that went unaccounted for by the AMD algorithm. More specifically, it would be beneficial if the AMD algorithm of an AMD system enabled users to optionally record any additional medicament deliveries, activities, or other interactions that may affect the AMD algorithm adaptation and incorporate such modified records in its future iterations of the AMD algorithm adaptation cycle.

The following examples describe beneficial AMD systems and AMD algorithms that a process to detect potentially missed meals for users and enable users to indicate that they ingested these meals even if they had initially missed entering those meals.

The following examples describe beneficial AMD systems and AMD algorithms that enable users to indicate additional insulin deliveries that may not be otherwise known or inferred by the AMD algorithm and allow the AMD algorithm to modify its behaviors accordingly and ensure its recommended insulin delivery is up-to-date and effective.

The following examples describe beneficial AMD systems and AMD algorithms that enable users to inform the AMD system following completion of manual insulin delivery, and for the AMD systems to effectively respond to those user interactions.

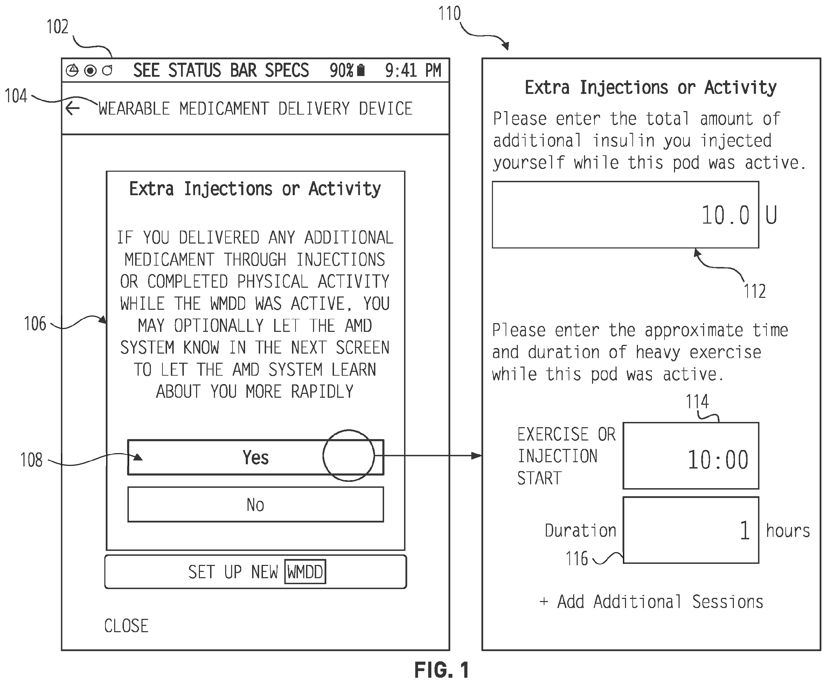

FIG. 1 illustrates an exemplary user interface that enables users to enter unaccounted event information that occurred during a prior adaptation cycle according to embodiments of the disclosed subject matter.

In an example experience for a diabetic user, a typical AMD system may generate a user history that incorporates insulin delivery history, instances of activity, and other records of factors that impact glucose and insulin dynamics to adapt their insulin deliveries to each user.

In contrast to the typical AMD system, the enhanced AMD system described herein may inquire whether the user, in the past (i.e., before the current or present time), may have participated in or been subject to some unaccounted [for] event (e.g., incurred additional medicament or participated in physical activity (e.g., impromptu physical exertion, such as changing a car tire, chasing their dog, a pick up game of basketball, or the like) or was subject to some other kind of unaccounted event (e.g., unscheduled meal or beverages) during the current period of assessment. Unaccounted events can happen, for example, when a user's wearable medicament delivery device has expired (e.g., expended all of the medicament), or became inoperable (e.g., lost power, was damaged, experienced an operational error (e.g., occlusion, fluid pathway blockage, or the like) without warning.

In an example embodiment, the user may enter the unaccounted event (i.e., unaccounted event information) at the end of each current period of assessment, such as the end of one pump session, as shown in FIG. 1. A current period of assessment (e.g., one pump session) may be a backward looking review of the past lifecycle of the wearable medicament delivery device. For example, a user may have begun using their current wearable medicament delivery device on Monday and the present day is Thursday and the user is replacing their current wearable medicament delivery device. The user may reflect back on the time from initial use on Monday of the current wearable medicament delivery device until Thursday. The period of time from Monday to Thursday may be considered the "current period of assessment" or the pump session. Of course, the number of days or number of hours may vary depending upon the user, the wearable medicament delivery device, and other factors (e.g., type of medicament used, or the like).

The "current period of assessment" may also correspond to an "adaptation cycle," which may be: 1) a lifecycle of a medicament delivery device; 2) a process executed by an automated medicament delivery algorithm when a wearable medicament delivery device is activated for use; or 3) both. The terms "current period of assessment" and "adaptation cycle" may be used interchangeably throughout the specification. The term "lifecycle of the medicament delivery device" may specifically refer to the operational lifespan of the device, beginning when the device is first prepared for use and ending when the device or a disposable component thereof is removed from the user.

An example AMD system that is configured to accommodate such unaccounted events may, as shown in FIG. 1, include a user interface 102, a new wearable medicament delivery device set up page 104, an unaccounted event prompt 106, an unaccounted event confirmation 108, an unaccounted event input 110, an extraneous medicament delivery amount 112, an extraneous exercise start 114, and an extraneous exercise duration 116.

The user interface 102 may be presented on a touchscreen display of a mobile device (not shown in this example). In an example, the mobile device may be a controller in an AMD system as described in a later example. One of the pages presented in the user interface 102 may be the new wearable medicament delivery device set up page 104. While setting up the new wearable medicament delivery device (labeled "WMDD") via the new wearable medicament delivery device set up page 104, the user interface 102 may present unaccounted event prompt 106. The unaccounted event prompt 106 may include language such as that shown in FIG. 1. If the user chooses to include information related to an unaccounted event (i.e., unaccounted event information), the user may select the unaccounted event confirmation 108. Alternatively, if all events were accounted for, the user may select the "NO" button (as the unaccounted event confirmation 108) to confirm that there is no unaccounted event information.

In response to a "Yes" selection from the unaccounted event confirmation 108, a new page may be presented, such as unaccounted event input 110, into which the user may input details of the unaccounted event information, such as extraneous medicament delivery amount 112, extraneous exercise start 114, extraneous exercise duration 116, or the like.

In an alternate embodiment, after entry of a value at the extraneous exercise start 114 and extraneous exercise duration 116, a processor may be operable to evaluate the period and the duration of the exercise by comparing them against existing history of activities, and if a user has previously entered or has an activity history that is different from the extraneous exercise that the user has currently entered, the existing history of activities may be modified to match the user's prior entries. Using the additional unaccounted event information, an AMD system that incorporates patterns of the user's activities to predict the user's future activities may adjust its pattern estimations (i.e., predictions) based on the corrected activity histories.

In a further example, it may be helpful to enable entry of a time and date by the user when the unaccounted event for medicament was delivered. For example, in the case, where the unaccounted event for medicament was insulin, the time and date perhaps input into a field adjacent to extraneous medicament delivery amount 112 enables the AMD algorithm to revise insulin delivery parameters useful in a diabetes treatment program, such as the estimate for insulin onboard (IOB) or the like. It would also be helpful in cases where the medicament is GLP-1 so the user does not deliver medicament when it may not be necessary to achieve the user's goals.

The unaccounted event prompt 106 may be presented on the user interface 102 at different periods, such as periodically, such as daily or hourly, whenever a wearable medicament delivery device is being replaced, after every meal, prior to shutdown of a controller device (e.g., a personal monitoring device, a smartphone, a computing device, a gaming device, or the like), or the like. The frequency or timing of the presentation of the unaccounted event prompt 106 may be controllable via a user settings menu or user preferences menu of the controller.

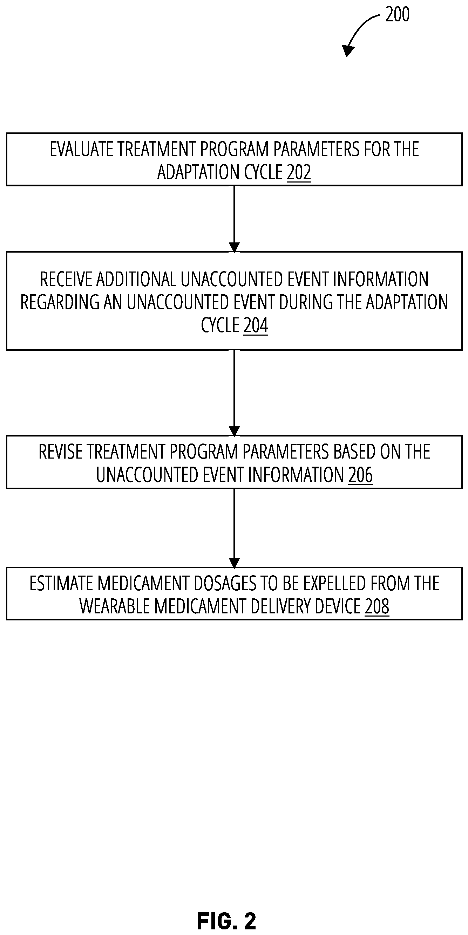

The AMD algorithm may be operable to take various steps in response to these inputs. FIG. 2 illustrates an exemplary process for obtaining unaccounted event information that occurred during a prior adaptation cycle according to embodiments of the disclosed subject matter.

Process 200 outlines the steps that may be used by an AMD algorithm to obtain and evaluate the unaccounted event information.

In block 202, process 200 evaluates treatment program parameters for the adaptation cycle. "Treatment program parameters" may refer to sensor values, AMD algorithm settings and weightings, such as total daily dosage (TDD), total daily insulin (TDI), target glucose measurement values or ranges, insulin on board, drug sensitivity, carbohydrate calculations, carbohydrate ratio, and the like. A target glucose measurement range is a predefined interval of glucose concentration values within which a patient's blood glucose level is intended to be maintained, e.g., ± 20 mg/dL around a target glucose measurement value such as 120 mg/dL.

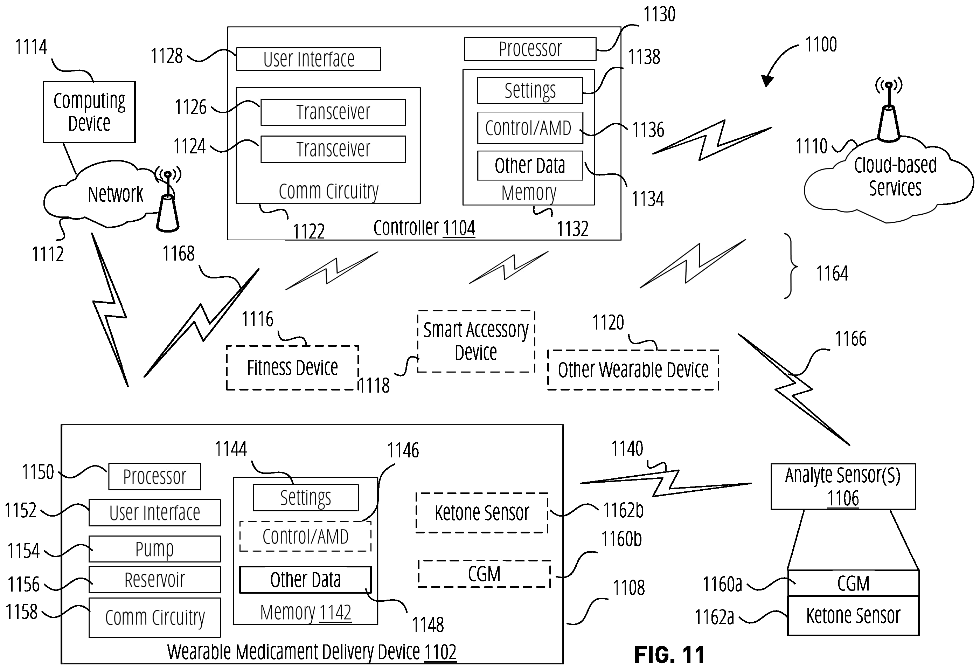

In an example, an adaptation cycle is a lifecycle of a medicament delivery device, such as a wearable medicament delivery device (WMDD) shown in the example of FIG. 11. Alternatively, or additionally, the adaptation cycle is a process executed by an automated medicament delivery algorithm when a wearable medicament delivery device is activated for use. For example, a user may replace their wearable medicament delivery device and position the replacement wearable medicament delivery device on their skin and activate it for use, whereby the AMD system begins determining when a next medicament dosage is to be delivered and which AMD algorithm operating parameters are to be updated from initial (or default settings). Initial or default settings may be those settings that are set prior to the first use of a replacement medicament delivery device.

In block 204, process 200 may receive, for example, via a user interface presented on a touchscreen display, additional unaccounted event information regarding an unaccounted event during the adaptation cycle. An unaccounted event may include, for example, at least one of an extraneous medicament delivery or omitted physical activity. An example of an extraneous medicament delivery may be an amount of medicament delivered via a device other than the wearable medicament delivery device. For example, a user's wearable medicament delivery device may fail or be otherwise inoperable, and the user may need a manually delivered bolus dosage to offset a meal.

An example of the omitted physical activity may be at least one of: exercise, or unplanned physical exertion. Unplanned physical exertion may be an unexpected physical event, such as changing a flat tire on their automobile, an extra trip on their bicycle, having to run to catch a bus, a pickup basketball game, or the like. Exercise can be exactly that, a run, weight training class, yoga class, bike ride, or the like. For example, the user may have a rest day in their training regimen, but may add an extra day of exercise to, for example, attain a fitness goal. The unaccounted event information may be the number of units (U) of medicament that was delivered, a date and time of the delivered number of units, or both.

In block 206, the process 200 revises treatment program parameters based on the unaccounted event information. In an example, the AMD algorithm may use the inputted unaccounted event information to update information such as TDD and carbohydrate consumed, remaining carbohydrate (also referred to as carbohydrate on board), or the like.

As part of the process 200 at block 208, the AMD algorithm may estimate medicament dosages (e.g., doses of insulin, or the like) to be expelled from the wearable medicament delivery device using, for example, the revised treatment program parameters.

Further, once a user enters values related to the unaccounted event information, the AMD algorithm may incorporate these parameters into further modifications of periodic adaptivity schemes of the AMD algorithm.

With regard to nomenclature in the following equations and discussion, total daily dosage (TDD) is an amount of insulin that a user actually received during the day (i.e., 24 hour period), while TDI is an estimate of the amount of medicament that a user is going to need on a daily basis in the future and is calculated using the TDD for the current day and the previous day's estimated TDI (i.e., *TDI_{old}*). The AMD algorithm may or may not use TDD and TDI interchangeably depending upon the circumstances.

Typically, the standard adaptation of the system may depend on the user's previous insulin delivery history, as follows: ${TDI}_{new} = \left(1-0.2⋅{N}_{days}\right) {TDI}_{old} + 0.2 ⋅ {N}_{days} ⋅ {TDD}_{new}$ Equation 1 where *N_{days}* is the number of days in the user history, the factor *TDI_{old}* is the user's previous TDI, and the factor *TDD_{new}* may be calculated as shown in Equation 2 shown below: ${TDD}_{new} = \frac{{\sum }_{k = 1}^{{k}_{end}} I \left(j-k\right)}{288 ∗ {N}_{days}}$ Equation 2.

In Equations 1 and 2 above, the new TDI, *TDI_{new}*, may be calculated by a weighted sum of the user's previous TDI, *TDI_{old}*, and the average daily dose for the newly available insulin history, or *TDD_{new}*. The weighting, i.e., the "0.2", may be tunable. The newly available insulin history *TDD_{new}*, as shown in Equation 2, may be calculated as the sum of all new insulin delivery history, divided by the number of 5-minute cycles in the newly available history, where 288 equals the number of 5-minute intervals there are in 24 hours, and *N_{days}* is the number of days (i.e., 24-hour periods) in the user history. Other interval lengths may also be considered.

In a further example embodiment as quantified in Equation 3 below, the *TDD_{new1}* may be augmented by user entries of unaccounted events, such as physical activity, collectively called "exercise," and additional injections, as follows: ${TDD}_{new 1} = \frac{{\sum }_{k = 1}^{{k}_{end}} I \left(j-k\right) + {I}_{extra , new} + 0.1 {\sum }_{q = 1}^{{q}_{end}} {d}_{q , exercise}}{288 ∗ {N}_{days}}$ Equation 3 where *TDD_{new1}* is a revised total daily dosage, the unaccounted for physical activity is quantified by the summation of *d_{q,exercise}* over the duration q, the unaccounted for insulin (or other medicament) is quantified by *I_{extra,new}*, and the insulin (or other medicament) delivered during the number of operational cycles that occurred within the *N_{days}* of the user history.

In Equation 3, the sum (i.e., amount of insulin delivered) of newly available insulin history may be increased by the amount of additional insulin that the user states as being delivered during the most recent adaptation period (i.e., *N_{days}*).

Further, an additional increase may be applied per the total duration of each unaccounted for exercise session (i.e., unaccounted for event or unaccounted for physical activity) reported by the user by applying a correlating factor, such as 0.1 units (U) per 1 hour of exercise. This additional increase in unaccounted for physical activity is represented by the summation of *d_{q,exercise}* from *q*=1 to *q_{end}*. The *d* may be referred to as the "delta" for the physical activity. While the example may use 0.1 U per 1 hour, other correlating factors may be used depending upon the user's physiology, or the like.

This increase attributable to the unaccounted for exercise may be applied given that these exercise sessions may not be a regular occurrence, and the insulin history in such cases may have *under* reported (or estimated) the user's insulin needs if such exercise sessions did not occur. Hence, the additional medicament due the extraneous medicament deliveries (i.e., added insulin from the unaccounted for insulin deliveries) and the unaccounted for physical activities, or exercise sessions, may cause the total daily dose of medicament to be increased. These extraneous medicament deliveries and/or unaccounted for insulin deliveries may be included in the "unaccounted event information" used by the process 200 and others.

Alternatively, the period and duration of these unaccounted for exercise sessions may also be compared against existing history of activities, and if users enter a different activity history, the existing history of activities may be modified to match the user's prior entries. An AMD system that incorporates patterns of the user's activities to predict the user's future activities may adjust its pattern estimations based on the corrected, or updated, activity histories.

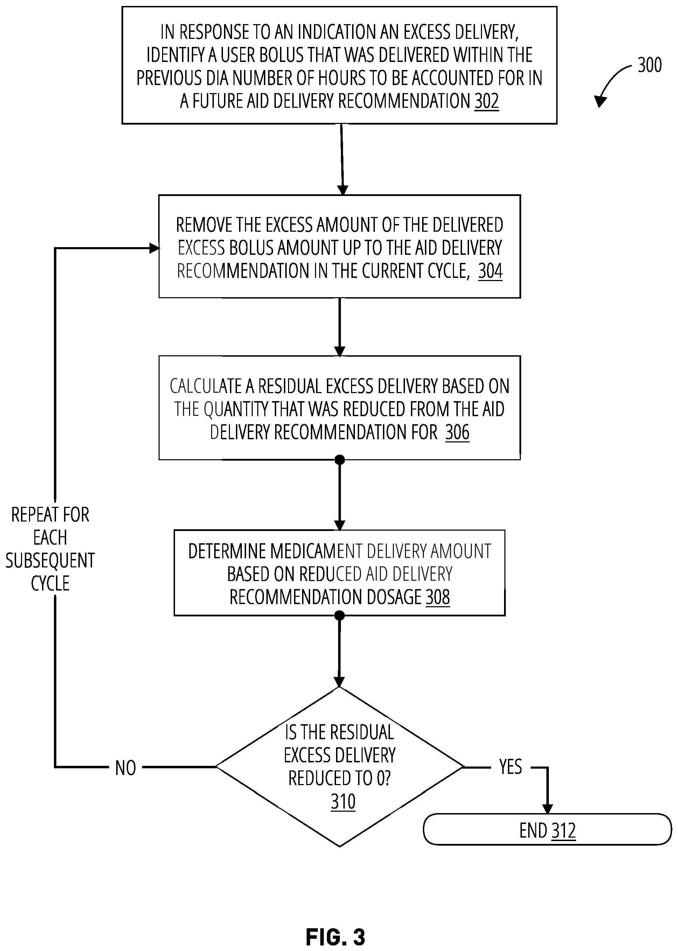

FIG. 3 illustrates another exemplary process for enabling users to provide feedback related to erroneous manual insulin deliveries according to the disclosed subject matter.

The disclosed subject matter also envisions situations in which a user erroneously boluses too much (or relatedly, entering too many carbohydrates when bolusing for a meal), and later realizing they entered too much (or accepted an erroneously high bolus amount as suggested by the bolus calculator based on the erroneously entered carbohydrate amount). In an example, there may be a situation where the user thinks they are going to eat a large meal, boluses for a large meal, but ends up eating a small meal, or not finishing their meal. This is the inverse of the example of FIG. 2 where the user can enter additional insulin delivered outside of the pump (e.g., via an insulin pen).

FIG. 3 illustrates an example erroneous manual delivery process 300 for informing the AMD system following completion of manual insulin delivery, and for AMD systems to effectively respond to those user interactions. Although the example erroneous manual delivery process 300 depicts a particular sequence of operations, the sequence may be altered without departing from the scope of the present disclosure. For example, some of the operations depicted may be performed in parallel or in a different sequence that does not materially affect the function of the erroneous manual delivery process 300. In other examples, different components of an example device or system that implements the erroneous manual delivery process 300 may perform functions at substantially the same time or in a specific sequence.

According to some examples, the erroneous manual delivery process 300 includes in response to an indication an excess bolus delivery, identify a user bolus that was delivered within the previous duration of insulin action (DIA) number of hours to be accounted for during a calculation of a future AMD system delivery at step 302. For example, a user indicates an excess delivery, and the system identifies a user bolus that was delivered within the previous DIA number of hours. The excess insulin that needs to be taken out of a future AMD system delivery is calculated. In an example, if the user had delivered 3U of bolus at 5 PM. The user triggers this excess delivery alert, via an input into a user interface at 5:30 PM. The excess delivery is calculated as 1.5U, for example.

According to some examples, the AMD algorithm calculates a modified recommended dosage by subtracting the excess amount of the bolus delivered from the AMD system delivery recommendation in the current cycle at step 304. The amount subtracted may be up to the amount of AMD algorithm delivery recommendation in the current cycle. In a specific example of step 304, the AMD algorithm recommends 0.1 units (U). The excess amount delivered may be, for example, 1.5U. In the erroneous manual delivery process 300, the AMD algorithm may use a preset reduction value, which may be a calculated value based on a user's physiological attributes such as insulin sensitivity, a time of day, or the like, or may be a preset value selected based on clinical data or a conservative treatment program. Since the excess amount delivered is 1.5U, which is greater than the recommendation, the AMD algorithm's recommendation is reduced by the preset reduction value, which may be, for example, 0.1U. In this example, the AMD algorithm delivery recommendation of 0.1U drops to 0, in which case there would not be a delivery of medicament for this current AMD cycle. An AMD cycle may be an operational cycle in which a sensor value is received, a medicament dosage is calculated based on the received sensor value, and the medicament dosage is delivered to a user. Alternatively, an AMD cycle may be an adaptation cycle.

According to some examples, at step 306, a processor executing the AMD algorithm calculates a residual excess delivery based on the quantity that was reduced from the AMD delivery recommendation in step 304. The residual excess delivery is recalculated based on the quantity that was reduced (e.g., the 0.1U) from the AMD algorithm's initial recommendation. As 0.1U was subtracted/removed from the AMD algorithm's recommendation at step 304, the residual excess delivery is, in this example, 1.5U - 0.1U, or 1.4U. In cases, where the AMD delivery recommendation and the present reduction value are equal, or substantially equal, no medicament delivery is made.

According to some examples, the method includes determining if the residual excess delivery amount has been reduced to 0U at decision block 310. In this example, the residual excess delivery amount is 1.4U. So, at decision block 310, the decision is NO, the residual excess delivery has not been reduced to 0U, so the erroneous manual delivery process 300 returns to step 304 for the subsequent cycle, i.e., AMD cycle.

Alternatively, if at decision block 310, the decision is YES, the residual excess delivery has been reduced to a residual excess delivery threshold value, such as, for example, 0 units (U), the erroneous manual delivery process 300 proceeds to done block 312. At done block 312, the system may initiate delivery of the AMD delivery recommendation dosage, or remainder thereof. A remainder thereof may be the AMD delivery recommendation dosage for the current cycle but reduced by the set 0.1U. If the difference between the AMD delivery recommendation dosage and the present reduction value is zero (0), a medicament delivery is not initiated for this cycle.

In a more detailed discussion of a typical example, users of AMD systems may augment automated insulin delivery by such systems with additional manual insulin delivery. In such embodiments, the user may request insulin delivery based on exogenous carbohydrate ingestion, desire for additional compensation for glucose excursion, or both, with the quantity typically defined by the following equation: $b \left(k\right) = {b}_{corr} \left(k\right) + {b}_{meal} \left(k\right)$ Equation 4 where *b*(*k*) is a bolus in the k^{th} cycle, *b_{corr}*(*k*) is a correction portion of the bolus, and bₘₑₐₗ(*k*) is a meal portion of the bolus.

The correction portion of the bolus b_{corr}(k) may be determined according to Equation 5: ${b}_{corr} \left(k\right) = max \left({IOB}_{req}\left(k\right)-{IOB}_{meal}\left(k\right)-{IOB}_{corr}\left(k\right),0\right)$ Equation 5 where *IOB_{req}*(k) is the insulin on board (IOB) required to bring the user's blood glucose to their target blood glucose, *IOBₘₑₐₗ*(k) is a meal portion of the user's current IOB (from prior meal boluses), and *IOB_{corr}*(k) is a correction portion of the user's current IOB (from prior correction boluses).

The IOB required to bring the user's blood glucose to their target blood glucose, IOB_{req}(k), may be calculated using the Equation 6: ${IOB}_{req} \left(k\right) = \frac{G \left(k\right) - SP \left(k\right)}{CF \left(k\right)}$ Equation 6 where *G(k)* is the current glucose concentration, *SP*(*k*) is the current glucose target (i.e., set point), and *CF*(*k*) is a correction factor (e.g., insulin sensitivity value, or the like).

Meanwhile, *Bₘₑₐₗ*(*k*) may be calculated using Equation 7: ${b}_{meal} \left(k\right) = \frac{CHO \left(k\right)}{IC \left(k\right)} - {IOB}_{corr , res} \left(k\right)$ Equation 7 where *CHOC*(*k*) is the carbohydrate in a meal (in grams) and *IC*(*k*) is the insulin to carbohydrate ratio of the user.

The residual correction IOB, if the correction bolus is zero (0U) after taking into account prior meal and correction IOB may be calculated according to Equation 8: ${IOB}_{corr , res} \left(k\right) = \left\{\begin{matrix}{IOB}_{corr} \left(k\right) - max \left(\frac{G \left(k\right) - SP \left(k\right)}{CF \left(k\right)}-{IOB}_{meal}\left(k\right),0\right) & {IOB}_{req} \left(k\right) - {IOB}_{metal} \left(k\right) - {IOB}_{corr} \left(k\right) \leq 0 \\ 0 & otherwise\end{matrix}\right.$ Equation 8

In Equation 8, the term *IOB_{corr,res}*(*k*), when *IOB_{req}*(*k*) *- IOBₘₑₐₗ*(*k*) *- IOB_{corr}*(*k*) is less than or equal to zero (0), has a value of the difference between *IOBcorr* and a maximum value of either *IOBreq*(k) or zero (0). Alternatively, when *IOB_{req}*(*k*) *- IOBₘₑₐₗ*(*k*) *- IOB_{corr}*(*k*) is greater than zero (0), the term *IOB_{corr,res}*(*k*) is zero (0).

In such embodiments, the user may request insulin delivery through the AMD system pump, and the algorithm within the pump may then account for the additional insulin being delivered by the user's bolus, under the assumption that the quantity of insulin matches the external factor that the user has compensated for that insulin (such as meal ingestion).

However, the user may post hoc become aware of an accidental over-delivery of insulin, such as due to an over-estimation of the amount of carbohydrate, or a new factor in reduced insulin needs that the user was not previously aware (such as a sudden exercise event). In such cases, the AMD algorithm may be unaware of the over-delivery of insulin and may only start responding to the faster than expected reduction in glucose.

In this example, the user may proactively inform the AMD algorithm as soon as the user is aware of the accidental over-delivery of medicament, enabling a more rapid response by the AMD algorithm as needed.

In the simplest example, the AMD system may simply allow the user to indicate *any* instance in which the user recognizes they had a manual over-delivery of insulin. Once the user indicates this instance, the AMD system may modify its estimate of the effect of insulin due to the user's most recent manual bolus.

For example, if the user's manual bolus was executed earlier than a number of hours equal to the user's current duration of insulin action (DIA) settings, the system may acknowledge the user's indication but may not modify its behaviors, given that the effect of such overdelivery may likely have been already taken into account and reflected in the user's current state.

If there was a recent manual bolus associated with this indication from the user within the current DIA, the algorithm may conservatively determine that a fixed percentage, such as 50%, or a value with a range, such as 40%-60%, 52%-57%, or the like, of the user's bolus may have been an overdelivery, and is taken into account by the AMD algorithm in subsequent AMD dosage recommendation.

In an alternative example, the portion of the overdelivery of a user's bolus may be applied as a factor that reduces future AMD insulin recommendations until the total amount of overdelivery is compensated. The determination of the factor may be represented as follows: ${I}_{excess} \left({k}_{trigger , N}\right) = 0.5 b \left({k}_{N}\right)$ ${I}_{excess , remaining} \left({k}_{trigger , N}\right) = {I}_{excess} \left({k}_{trigger , N}\right)$ ${I}_{excess , remaining} \left(k\right) = {I}_{excess , remaining} \left(k-2\right) - {I}_{excess , removed} \left(k-1\right)$ ${AMD}_{f} \left(k\right) = max \left(0,AMD\left(k\right)-{I}_{excess , remaining}\left(k-1\right)\right)$ ${I}_{excess , removed} \left(k\right) = AMD \left(k\right) - {AMD}_{f} \left(k\right)$ where the excess insulin delivered *I_{excess}* is calculated at the time the user triggers the indication (e.g., step 302 of FIG. 3) of the erroneous manual delivery, *k_{trigger,N}* based on, for example, an estimated 50% (the 0.5 multiplier, which can be parameterized or may be some other value, such as a value in the range of 40% - 60%) of the user's most recent bolus associated with the user's N^{th} trigger, *b*(*k_{N}*). The remaining insulin that needs to be taken out from future AMD insulin delivery recommendations is calculated by removing this initially calculated excess amount, by the proportion that was actually taken away in each cycle. The above equations may be used in erroneous manual delivery process 300.

As another alternative example, the user may optionally specify the estimated proportion P of excess delivery, such as 10%, 20%, 30%, or others. This may adjust the factor of 0.5 (i.e., the 50% of the estimated user's most recent bolus) to calculate the initial excess insulin delivered, as follows: ${I}_{excess} \left({k}_{trigger , N}\right) = P ⋅ b \left({k}_{N}\right) .$

As a further alternative, the user may be enabled to present specific quantity of increased insulin I_{excess,input} that was delivered, which may directly replace the excess insulin delivery calculation, as follows: ${I}_{excess} \left({k}_{trigger , N}\right) = {I}_{excess , input} .$

As a final embodiment, rather than adjusting the algorithm's insulin delivery recommendations, the insulin delivery history to calculate the insulin-on-board (IOB) may be augmented. Specifically, the recorded insulin delivery for IOB calculations in the cycle the user has provided the N^{th} indication, *k_{trigger,N}* may be augmented by this excess insulin delivery $calculation , {I}_{excess} \left({k}_{trigger , N}\right) .$

These different permutations of the *I_{excess}* parameter may be used in the erroneous manual delivery process 300. The erroneous manual delivery process 300 may be considered the inverse of the process 200 of FIG. 2 where the user can enter additional insulin delivered outside of the pump (e.g., via an insulin pen).

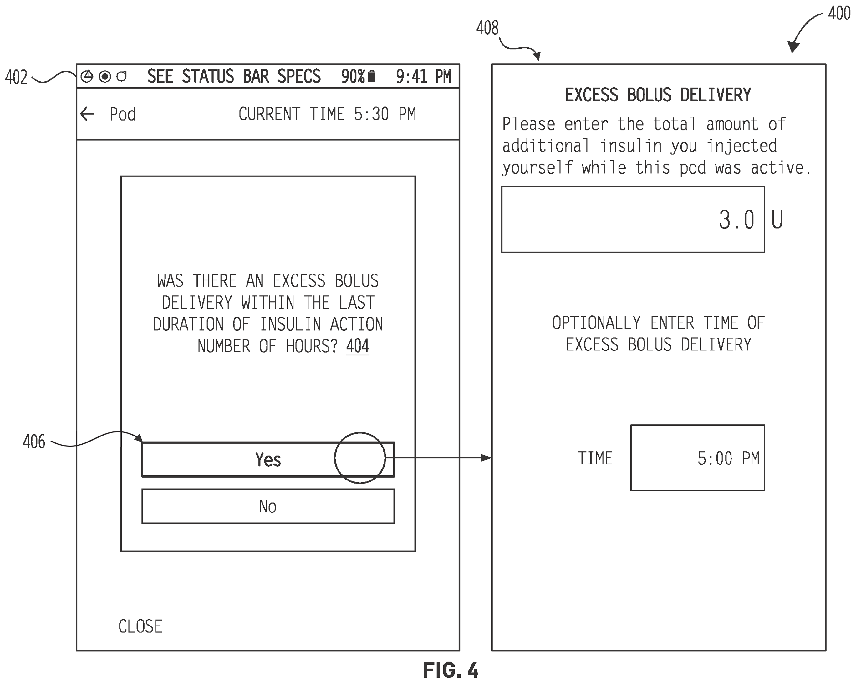

FIG. 4 illustrates an exemplary user interface according to the disclosed examples. Unaccounted events can happen, for example, when a user's wearable medicament delivery device has expired (e.g., expended all of the medicament), or became inoperable (e.g., lost power, was damaged, experienced an operational error (e.g., occlusion, fluid pathway blockage, or the like) without warning.

The excess bolus notification 400 may be user interface presented on a controller device of the AMD system. The excess bolus notification 400 includes an excess bolus prompt 404, a user interface 402, an excess bolus confirmation 406, and an excess bolus input 408. The excess bolus prompt 404 may be presented in response to an input, which may be via another window of user interface 402, to the controller that a bolus delivery was not provided to the AMD algorithm. Upon presentation of the excess bolus prompt 404 in the user interface 402, the user may indicate a YES in excess bolus confirmation 406. In response to the affirmative confirmation via excess bolus confirmation 406, the controller processor may present the excess bolus input 408, which is a window of the user interface 402 that enables details of the bolus to be entered and provided to the AMD algorithm.

From an earlier example, assume the user provides an indication of the excess bolus delivery to user interface at 5:30 PM. In response the controller processor may present excess bolus prompt 404, which the user confirms via excess bolus confirmation 406. In the excess bolus input 408, the user may input that 3U of bolus was delivered and optionally include that the excess delivery was given at 5 PM. Based on this information the AMD algorithm may react, for example, as described in erroneous manual delivery process 300 of FIG. 3.

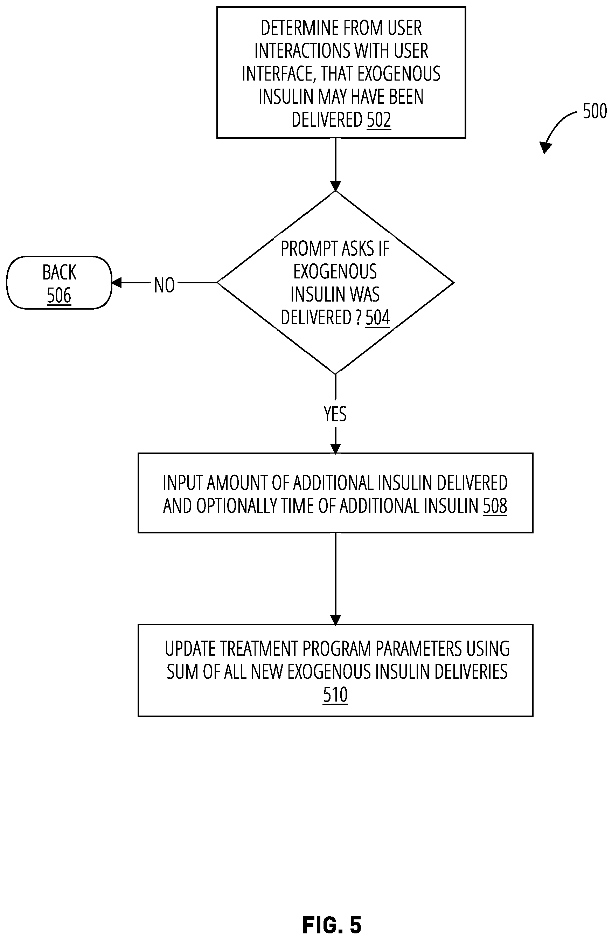

FIG. 5 illustrates an exemplary process for algorithmic responses to user entries of unknown insulin deliveries according to the disclosed subject matter.

In the following discussion of this example, an automated medicament delivery (AMD) system may modify its medicament delivery recommendations based on known previous medicament delivery histories. However, users may also deliver additional medicament at different times, such as delivering insulin beyond meal ingestions, such as via manual injections. In the example of insulin, when an AMD algorithm recommends insulin deliveries without knowledge of these additional manual insulin deliveries, erroneous insulin delivery and increased risk of hyperglycemia or hypoglycemia for the user may be the result of delivering the AMD algorithm-recommended insulin deliveries.

For example, if a user delivers exogenous insulin that is unknown to the AMD system, the functions of the AMD system may not accurately reflect the user's true insulin needs, leading to sub-optimal insulin delivery recommendations by the AMD algorithm.

The following discussion describes algorithms and processes to enable users to indicate, via a user interface, an additional medicament delivery to the AMD algorithm that may not otherwise be known by the AMD algorithm and allow the AMD algorithm to modify its behaviors accordingly and ensure its recommended medicament delivery is optimal.

In an example, the term "duration of insulin action (DIA)" may refer to the length of time that insulin remains active and available in the user's body after a correction or meal bolus. DIA may also be considered a decay rate of the delivered insulin as the length of time that the insulin is unused by the body or no longer effective is always decreasing. It is noted that DIA may vary by some degree from person to person.

In process 500, the AMD algorithm may be made aware of these exogenous insulin deliveries through user interactions, such as requesting delivery of less insulin, indicating that the user is feeling that their glucose is high or low, or the like. For example, the user can press a HIGH button presented on a user interface that is prompting the user for input related to how they are feeling. Based on the user interactions, the processor is operable to develop a probability from the user interactions with the user interface that exogenous insulin may have been delivered at step 502. For example, several indications within a predetermined time period via a user interface by the user that they are feeling that their blood glucose is "HIGH," may cause the AMD algorithm to develop a high probability (e.g., 60%, 70%, or higher) that the user may have delivered exogenous insulin without informing the AMD algorithm. As a result, the processor may generate a prompt at decision 504, inquiring if the exogenous insulin was delivered. If the response is NO at decision 504, the process returns back to monitoring the user interactions via end 506. Alternatively, if the response is YES at 504, the interaction exogenous delivery process 500 may proceed to step 508.

At step 508, the user may be presented with a user interface having an input field that receives a time when an exogenous insulin delivery that was not previously entered, for example, because the user forgot to enter it. This input field may be indicated on a user interface as "enter additional insulin," "missed reported insulin delivery," or any other terminology, such as shown in unaccounted event prompt 106.

In the example, while the AMD algorithm's short-term calculations, such as the insulin onboard for a current operational cycle *k*, which may be referred to as (*IOB*(*k*)), may not be impacted; however, at step 510, the AMD algorithm may be operable to adjust its estimate of the user's total daily insulin (*TDI*) requirements based on a sum of all new exogenous insulin deliveries entered by the user. For example, the *TDI* may be calculated for a day N, represented by *TDI_{N}* as follows: ${TDI}_{N} = \left(1-0.2⋅{n}_{data}\right) {TDI}_{N - 1} + 0.2 ⋅ {n}_{data} ⋅ {I}_{previous plus unaccounted}$ Equation 9 where ${I}_{previous pluse unaccounted} = \frac{{\sum }_{j = 1}^{{k}_{end . N}} I \left({k}_{end , N}-j\right) + {\sum }_{j = 1}^{{k}_{end . N}} {I}_{ex} \left({k}_{end , N}-j\right)}{{D}_{N}}$ Equation 10, and where the N^{th} estimate of the total daily insulin (*TDI_{N}*), where *N* is a day, may be a weighted sum of the previous estimate of the user's total daily insulin and the sum of all insulin delivery since the previous calculation, divided by the number of days *D_{N}* since the previous calculation, and weighted by the amount of new days of available history, *n_{data}*. The factor *Iₑₓ*(*k*) is the user's exogenous insulin delivery entry at the *k*^{th} operational cycle and the *k_{end,N}* is the operational cycle at the end of day *N*.

The foregoing example process provides users with the ability to revise their medicament delivery history to include unaccounted event information, which enables an AMD algorithm to modify its medicament delivery recommendations based on a now-known previous medicament delivery history (i.e., the revised medicament delivery history).

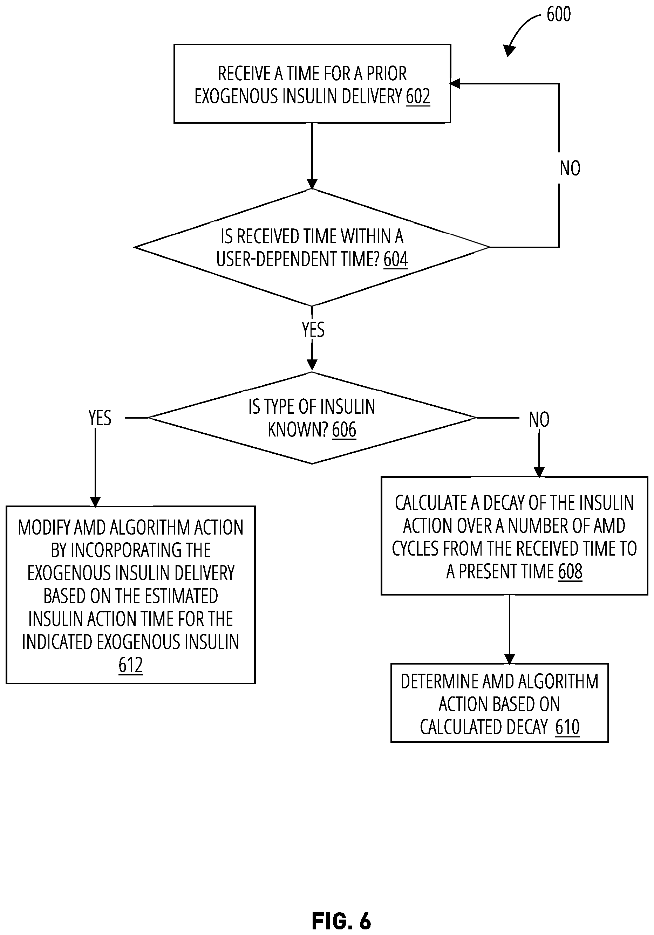

FIG. 6 illustrates another exemplary process for algorithmic responses to user entries of unknown insulin deliveries according to the disclosed subject matter.

In an alternative, or an optional addition, to the exemplary process of FIG. 5, the exemplary algorithm response process 600 may be implemented by the AMD algorithm via a user interface of a controller device. The algorithm response process 600 is operable to enable a user to enter a time for an exogenous insulin delivery within a user-dependent time, or preset time, such the last 4 hours, or the like (at step 602). In the example, the user-dependent time may be substantially equivalent to the duration of insulin action (DIA) (e.g., four (4) hours value of the DIA).

At decision step 604, the AMD algorithm may determine if the received time is within a user-dependent time. If the response is NO at decision step 604, the process returns to step 602 to wait for a time for a prior exogenous insulin delivery to be received. Alternatively, if the response is YES at decision step 604, the process may proceed to decision step 606. At decision step 606, the AMD algorithm may determine if the type of insulin is known. For example, the user may enter the type of insulin being used at time of setting up the wearable medicament delivery device, or the like. If the insulin type is unknown, a NO response at decision step 606, the AMD algorithm may be operable to modify its IOB calculations based on the inputted time of the exogenous insulin delivery being within, for example, the user-dependent time.

In a specific example, the AMD algorithm may modify the IOB calculation, such as the following: $\begin{matrix}IOB \left(k\right) = \\ \left(\left[I\left(k\right)-b\left(k\right) I\left(k-1\right)-b\left(k-1\right) … I\left(k-DIA\left(k\right)⋅12\right)-b\left(i-DIA\left(k\right)⋅12\right)\right]\right) \\ + \left[{I}_{ex}\left(k\right) {I}_{ex}\left(k-1\right) … {I}_{ex}\left(k-DIA\left(k\right)⋅12\right)\right] \times \\ \left[\begin{matrix}1 \\ 1 - \frac{1}{DIA \left(k\right) ⋅ 12} \\ 1 - \frac{2}{DIA \left(k\right) ⋅ 12} \\ ⋮ \\ \frac{1}{DIA \left(k\right) ⋅ 12}\end{matrix}\right]\end{matrix}$ Equation 11

The matrix at the end accounts for the incremental decay of the insulin action over the cycles in the 4 hours of the DIA. In clinical examples, DIA may be 4 hours, which means insulin is effective for reducing blood glucose in the body for 4 hours. In typical conditions, DIA may be user dependent as some users may have a DIA that is greater than 4 hours, and other persons may have DIAs that are shorter. Thus, DIA may be 4 hours, more than 4 hours (such as 5, 6, or 7 hours), or less than 4 hours (such as 2 or 3 hours). The AMD algorithm is operable to determine an AMD algorithm action based on the calculated decay at step 610.

Alternatively, since the user has indicated exogenous insulin delivery within the DIA value and the type of insulin delivered or delivery mechanism (e.g., pen, syringe, inhaler, or the like), via the user interface, the response at decision step 606 is a YES. The algorithm response process 600 proceeds to step 612, the AMD system may be operable to modify its incorporation of the exogenous insulin delivery based on the estimated insulin action time for the indicated exogenous insulin. An example of incorporation of this additional indication may be as follows:

**TABLE 1**

| **User-delivered additional Insulin Type within the previous DIA hours** | **AMD algorithm action** |
|---|---|
| Long acting Insulin | No change to IOB; add to TDI estimate |
| Subcutaneous insulin injection/pen: RAPID | Include within IOB calculation per the user's indicated timing of delivery |
| Ultra rapid acting insulin, such as inhaled insulin | Apply a fixed 2-hour DIA decay factor rather than the user's standard DIA decay factor |

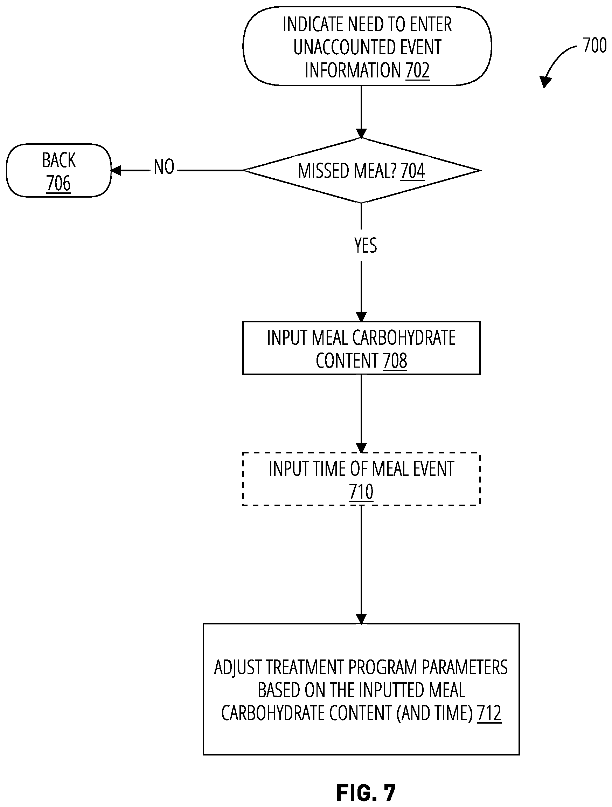

FIG. 7 illustrates an exemplary process that enables a user to indicate mistaken inputs related to carbohydrate ingestion according to the disclosed subject matter.

The following example provides processes implementable by a processor executing an AMD algorithm to detect potentially missed meals for users, and enable users to indicate, via a user interface, that they ingested these meals even if they had initially missed entering those meals into the AMD system.

Previously, AMD systems enabled users to indicate their meal ingestions as part of their insulin therapy. However, due to the reliance on user's manual input of these meals, there is a significant likelihood of incorrect or missed inputs, which both hamper the glucose control outcomes of these users and reduce the ability of AMD systems to adapt to the users' insulin needs.

In an example of an improved AMD system, in which a user who missed entering a meal (i.e., a missed meal) into the AMD system, may be presented with a prompt, but instead of extra injections or activity as in unaccounted event prompt 106, the AMD algorithm may cause presentation of a prompt for entering information regarding the missed meal. For example, the AMD algorithm may generate the prompt on a user interface on a control device, such as a smart phone, a guardian's or parent's smart phone, computer, a personal management device, or the like).

In a further example, an AMD system may enable users to also indicate when (time and/or date) they had ingested the meal, as well as the quantity of carbohydrate in the meal. Specifically, an AMD algorithm may dynamically respond to both the timing and quantity of carbohydrates that were indicated to be ingested by the user to modify a bolus dosage.

For example, the modified bolus dosage to compensate for the missed meal may be calculated using the following equation: ${B}_{mod} \left(k\right) = \frac{CHO \left(k\right)}{500 / TDI} + \frac{CGM \left(k\right) - SP \left(k\right)}{1800 / TDI} - IOB \left(k-min\left(\frac{D}{5}, 6\right)\right)$ Equation 12 where the modified bolus amount, *B_{mod}*(*k*), that is to be delivered in the current k^{th} cycle follows the typical calculation of boluses that divide the carbohydrate intake *CHO*(*k*) by the estimated insulin to carbohydrate ratio (i.e. 500/*TDI*), combining with the typical calculation for correction bolus (i.e., glucose *CGM*(*k*) minus target set point *SP*(*k*)) divided by the estimated correction factor (i.e., 1800/*TDI*). However, the insulin-on-board, or *IOB*, is adjusted to account for the bolus amount that the user should have received at the time the user had ingested the meal. This is represented by the value *D*, which equals the number of minutes prior to the time of bolus that the user may indicate the start of their meal ingestion. Equation 12 may also be adapted to use the value D in other units of time, i.e., seconds or hours. In the exemplary Equation 12, the duration of time that this historical assessment may review may be limited to no more than 30 minutes, or 6 cycles of the AMD system, as the AMD algorithm may have independently begun compensating for the glucose rise due to the meal ingestion.

In the short term (e.g., 1.5 - 3 hours), this may result in modification of current insulin-on-board estimates and other insulin delivery estimates of the algorithm. In the present example, the additional insulin delivery due to a rise in glucose by the AMD system between the time that the user indicated the meal and the current cycle may be considered an insulin delivery in response to ingestion of a meal, rather than glucose excursions away from the set point.

As mentioned above, when a user ingests a meal but misses informing the AMD algorithm of the meal, the AMD algorithm may respond to the increased glucose caused by the ingested meal as a typical deviation or excursion of the blood glucose. Hence, the AMD algorithm may begin delivering insulin to merely return the glucose to the target set point without attempting to compensate for the unaccounted-for carbohydrates. In other words, the AMD algorithm is responding to a glucose excursion, and not ingestion of a meal.

However, by being able to update the AMD algorithm of the missed meal, when the user informs the AMD system of the missed meal after the AMD system has begun responding to the rise in glucose, the AMD system may alter medicament delivery safety constraints with respect to insulin delivery by considering the insulin delivered (for example, in the last 30 minutes) as compensation for a meal, instead of being delivered to address a glucose excursion.

For example, an AMD algorithm now provided with the unaccounted event information regarding the missed meal (i.e., past meal information) may, based on this information, modify a number of short-term AMD algorithm settings, such as medicament delivery safety constraints, such as IOB calculations, integral constraints (such as a setting for delivering a percentage % of TDI within a set time period, e.g., 3 hours, without a meal), and others.

Additionally, overall estimates of the user's basal versus bolus needs made by the AMD algorithm may also be modified. For example, the AMD system may, for instance, assume the user needs less insulin delivery from the AMD algorithm than expected as part of the basal delivery, than should be delivered by the user's manual bolus. The AMD system may also, or alternatively, cause a varying penalization of current insulin delivery based on past basal versus bolus insulin history. In yet a further alternative, the AMD system may make implementations, such as the insulin deliveries for manual bolus and/or meal compensations may be treated differently than automated and/or correction compensations in AMD system. Specifically, an AMD algorithm may observe that a user may require a certain amount of insulin to compensate for meal ingestions and other disturbances in their glucose management. The insulin that may be required to compensate for manual boluses and meal compensation may vary depending on the user's overall meal ingestion each day and may not be considered as part of their average insulin requirements outside of meals, which would be instead dependent on the user's insulin sensitivities.

The previous example covered instances where the user, via a user interface, informed the AMD system of a missed meal a relatively short time after the meal, such as 30 minutes, 45 minutes, 60 minutes, or the like. But, in a further example, the AMD algorithm may enable users to indicate, via the user interface, a missed meal a significant time beyond the near-term periods (e.g., 30 minutes, 45 minutes, 60 minutes), when insulin delivery has already occurred. For instance, a user may record a meal ingestion more than 4, 6, or 8 hours prior to the current cycle that was missed and be fully compensated by the AMD algorithm. Then, the AMD algorithm may also re-classify additional insulin that was delivered by the system beyond basal for a period of time as meal insulin requirements rather than glucose excursion (correction) insulin requirements. The period of time may be 90 minutes, 120 minutes, 2.5 hours, or the like (e.g. anywhere between 30 minutes to 12 hours prior, more specifically 90 minutes to 8 hours prior), after that indicated meal event, with a maximum amount corresponding to the indicated meal carb content. The re-classification of the additional insulin may impact long term estimates made by the AMD algorithm for the user's insulin needs, such as basal versus bolus insulin needs, and other factors, because the AMD algorithm can now account for insulin delivery as being related to a meal as opposed to a glucose excursion.

In an operational example, FIG. 7 illustrates an example missed meal update process 700 for adjusting AMD algorithm parameter, medicament delivery dosages, and medicament delivery times. Although the example missed meal update process 700 depicts a particular sequence of operations, the sequence may be altered without departing from the scope of the present disclosure. For example, some of the operations depicted may be performed in parallel or in a different sequence that does not materially affect the function of the missed meal update process 700. In other examples, different components of an example device or system that implements the missed meal update process 700 may perform functions at substantially the same time or in a specific sequence.

As shown in FIG. 7, the AMD algorithm may implement a missed meal update process 700. For example, a user may indicate a need to enter a missed meal (e.g., a need to enter unaccounted event information) at start 702. The process 700 may present at a prompt for a decision as to whether the missed meal prompt 704, which may ask for confirmation that a meal was missed. If the user inputs NO, a meal was not missed for entry into the AMD algorithm, the process 700 may proceed back to the prior window of the user interface via block 706. However, if the response is Yes, in response to missed meal prompt 704, the process 700 may proceed to enabling the user to input the meal carbohydrate content at step 708. Additionally, the user interface may query the user for a time of the missed meal event at block 710. The processor may receive the unaccounted event information regarding the missed meal, and be operable to evaluate the received unaccounted event information (e.g., the meal carbohydrate content and, time if entered). More specifically, based on the inputted meal carbohydrate content (and the time of the missed meal event, if entered), the AMD algorithm may be operable to adjust treatment program parameters for the user, such as insulin on board amount, recommended delivery dosages, and other estimates based on the inputted meal carbohydrate content (and time) at step 712. For example, the processor is operable to, based on a result of the evaluation, determine whether to adjust one or more of: a bolus amount, an amount of insulin on board, or a maximum amount of insulin to deliver in a next operational cycle. Further examples and details of the adjustments to the treatment program parameters are described throughout the specification.

The AMD system may be operable to implement changes based on the modifications to the respective parameters. For example, a revised TDI may be used immediately in the calculation of a basal dosage or a modified bolus dosage may be applied immediately. In other examples, the use of the revise parameters may not be used immediately as some calculations, such as adaptation cycle calculations, are not performed until a certain amount of time has passed or an event, such as replacement of a wearable medicament delivery device (WMDD) has occurred. For example, the proportion of medicament delivered as basal with respect to bolus may be altered depending, for example, upon how recent the carbohydrates or insulin was delivered. For example, the AMD algorithm may be providing the user with 20U via basal delivery and 30 U of insulin by bolus delivery. But the user may indicate they took 3 U of insulin as a bolus delivery to compensate for a meal consumed about 6 hours ago, so the AMD algorithm may modify the proportion of basal delivery to bolus delivery from, for example, 20/30 U to 23/27 U.

These AMD algorithm modifications may be incorporated almost immediately and may affect calculations by the AMD system with respect, for example, to basal medicament delivery. Alternatively, the AMD algorithm modifications may be held for implementation, for example, at a next adaptation cycle, when a large amount of data is processed and utilized by the AMD algorithm. In either case, the calculations are performed depending upon when the carbohydrates are consumed or when the last dosage of insulin, either basal or bolus, was delivered.

In an operational example, a medicament delivery system that includes a wearable medicament delivery device, a processor, and a touchscreen. The processor may be operable to execute an AMD algorithm. In anticipation of consuming a meal, a diabetic user may enter an estimate of the expected carbohydrates in the meal. However, when the meal is served, the diabetic user realizes they underestimated the carbohydrate content of the meal. The processor may be operable to, after start of an extended bolus to compensate for a meal, present a user interface on the touchscreen display enabling entry of a revised carbohydrate amount for the meal. The presented user interface may be a bolus calculator modified to allow for modification of the previously inputted carbohydrate values. The processor, in response to the entry of the revised carbohydrate amount for the meal, may be operable to determine a remainder of the started extended bolus yet to be delivered. The processor may calculate an updated dosage for the started extended bolus and revise the remainder of the started extended bolus yet to be delivered based on the calculated updated dosage for the started extended bolus. In this example, since the diabetic user initially underestimated the carbohydrate content of the meal, the revised remainder is likely greater than the determined remainder of the started extended bolus yet to be delivered. The processor may cause the revised remainder to be expelled by the wearable medicament delivery device.

The following enables users to indicate that they had consumed a meal some time after the meal was ingested (i.e., there were carbohydrates that were missed from being input into the AMD algorithm). Being able to make up for this error is beneficial because with the updated missing carbohydrate information (e.g., unaccounted event information), the AMD algorithm may better compensate for the ingested meal by accounting now for the missing carbohydrate.

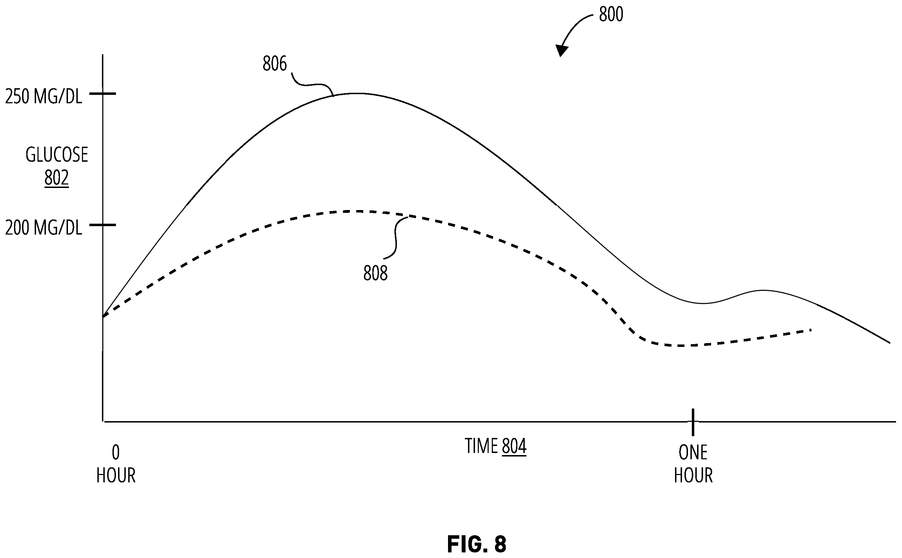

FIG. 8 illustrates a graph showing benefits of additional and/or on-time bolusing in response to a missed meal according to examples disclosed herein. Additionally, a user may be informed of potential improvements in therapy upon entry of past meal ingestion. Specifically, if the user enters a past meal ingestion, and their entered timing of the meal corresponds to a period of hyperglycemia, the user may be informed of beneficial effects of additional and/or on-time bolusing during this period.

For example, a simulation of the user's glucose traces if the user did deliver a bolus at the time may also be indicated. This may be calculated as shown in Equations 13 and 14 below, for example, by utilizing the user's glucose concentrations at the start of the indicated meal period, and recursively using the AMD algorithm to show predicted glucose concentrations: $G \left(k\right) = {b}_{1} G \left(k-1\right) + {b}_{2} G \left(k-2\right) + {b}_{3} G \left(k-3\right) + {KI}_{true} \left(k-3\right)$ Equation 13 where *G(k)* is the glucose reading for the *k*^{th} and future cycles associated with the user's meal ingestion, and *Iₜᵣᵤₑ* is the user's estimated insulin delivery if the user had delivered an appropriate bolus, such as the following: ${I}_{true} \left(k\right) = I \left(k\right) + \frac{{CHO}_{true} \left(k\right)}{500 / TDI} + \frac{CGM \left(k\right) - SP \left(k\right)}{1800 / TDI}$ Equation 14 where *CHOₜᵣᵤₑ* may be the past CHO entry amount for this meal that was entered by the user. As stated above, the AMD algorithm may be utilized recursively to show a new glucose trajectory, such as estimated glucose 808, over a fixed window, such as 1 hour, as shown in FIG. 8. The current glucose trajectory is shown as current glucose measurements 806.

A benefit of presenting the trajectory of the estimated glucose 808 is that it gives the user a visual indication (or prompt) of how much better maintained their glucose levels are (e.g., within range of their set points) if they remember to input all events, or otherwise account for all events that occur.

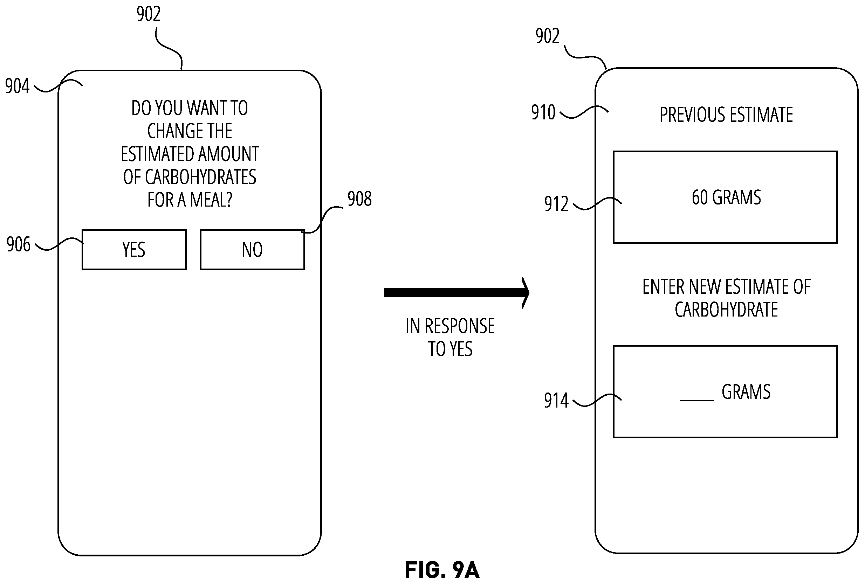

FIG. 9A illustrates a user interface for enabling updating of an initial carbohydrate estimate according to the disclosed subject matter.

A controller device of an AMD system executing an AMD algorithm and the exemplary processes described herein, may include a user device 902. The user device 902 has a user interface that is operable to present a meal adjust prompt 904. The meal adjust prompt 904 includes a query to the user, with confirmation buttons, a YES confirmation button 906, and a No confirmation button 908, for responding to the query. In response to YES confirmation button 906, the processor of the controller device is operable to present a user revision screen 910. Screen 910 is operable to present a previous estimate 912, and an interactive field including a new estimate input field 914. The new estimate input field 914 enables a user to enter a new estimate of the carbohydrate content of a meal, thereby updating the initial carbohydrate estimate of a meal, and enabling the AMD algorithm to update its treatment program parameters based on the updated carbohydrate estimate. Screen 910 may also display a time of when the previous estimate 912 was entered by the user. Screen 910 may also display a medicament amount that was estimated to be delivered, for example as a bolus, based on the previous estimate 912. Screen 910 may display a listing of prior carbohydrate entries by the user, the corresponding medicament amount to be (or that was) delivered based on the carbohydrate entries, the time when the user entries were entered, and (if applicable) the time when the medicament was delivered, or how much of the medicament has been delivered at the present time. Screen 910 may also display a new medicament amount that is estimated to be delivered based on the new estimate 914. Screen 910 may also display a differential value of the medicament based on the previous estimate 912 and the new estimate 914. The new estimate 914 may be higher or lower than the previous estimate 912. For example, a user may have believed that they would consume 60 grams of carbohydrates but then consumed only an estimated 30 grams of carbohydrates because the user had to cut the meal short, for example. Alternatively, the user may have believed that they would consume 40 grams of carbohydrates but then consumed an estimated 70 grams of carbohydrates because the dish they ordered at the restaurant turned out to be much larger than expected, for example.

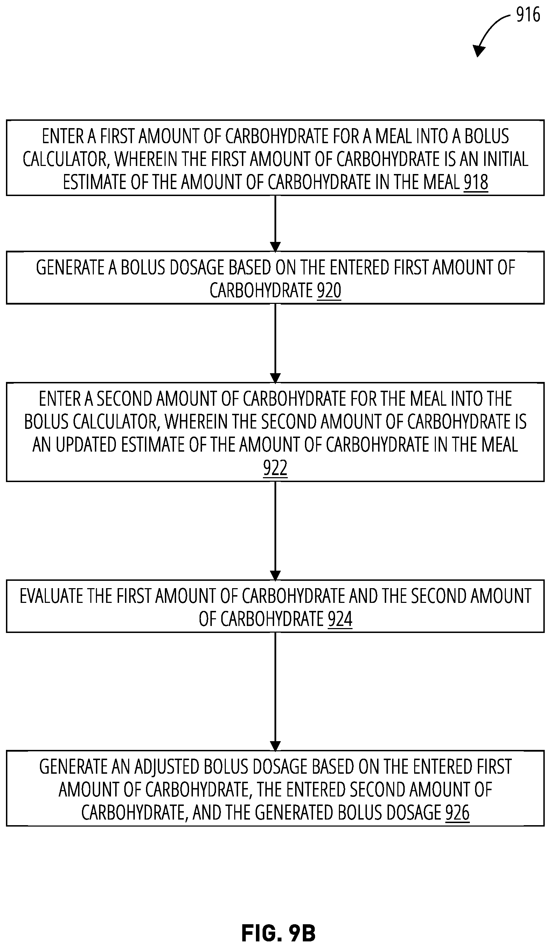

As shown in FIG. 9B, an exemplary process 916 may include a step where a user enters a first amount of carbohydrate (e.g., an amount of carbohydrates in grams) for a meal into a bolus calculator (step 918). The bolus calculator that may be executed by the AMD algorithm receives in the first carbohydrate amount for the meal. In this example, the first amount of carbohydrates for the meal may be an initial estimate of the amount of carbohydrates in the meal. In step 920, the bolus calculator may generate a bolus dosage based on the entered first amount of carbohydrates. The process 916, at step 922, may be operable to enable entering via a user interface a second amount of carbohydrate for the meal into the bolus calculator. In the example, the second amount of carbohydrate may be an updated estimate of the amount of carbohydrate in the meal. At step 924, a processor executing the AMD algorithm is operable to evaluate the first amount of carbohydrates and the second amount of carbohydrates. In step 926, process 916 generates an adjusted bolus dosage based on the entered first amount of carbohydrate, the entered second amount of carbohydrate, and the generated bolus dosage.

In a second proposed embodiment, the user may also indicate that they may have entered an incorrect amount of carbohydrate in earlier bolus requests. In this example, the AMD system may enable users to provide to a bolus calculator of the AMD system corrected carbohydrate amounts for a meal, and may modify the AMD algorithm behavior based on the direction of the incorrect carbohydrate entries, as follows: ${B}_{a} \left(k\right) = \left\{\begin{matrix}0 & {CHO}_{a} \left(k\right) < 0 \\ \frac{{CHO}_{a} \left(k\right)}{500 / TDI} - IOB \left(max\left({k}_{lastbolus}, \frac{D}{6}\right)\right) & {CHO}_{a} \left(k\right) > 0\end{matrix}\right.$ Equatiob 15 where *CHOₐ*(*k*) is the adjusted carbohydrate (which may be a positive value or negative value when considered with respect to the previously entered amount of carbohydrate, if the adjusted carbohydrate is less than previously entered carbohydrate, *CHOₐ*(*k*) has a negative value) and *k* is the current operational cycle, the variable *k_{lastbolus}* was the operational cycle when the last bolus was given, and factor *D*/6 is a time limit for when action is taken by the AMD algorithm. In the factor *D*/6, the *D* is the number of minutes since the meal was ingested, and the value "6" in the denominator is selected to represent the number of operational cycles *k* in 30 minutes (when each operation cycle is approximately 5 minutes in duration). In an example, the *CHOₐ*(*k*) may be negative when user realizes that the actual number of carbohydrates is less than what was input into the AMD system, and, conversely, *CHOₐ*(*k*) may be positive when the user realizes that the actual number of carbohydrates is more than the previously entered amount of carbohydrate input into the AMD system.

If the user delivered a bolus, i.e., a single dose of medicament all at one time, if the *CHOₐ* is less than zero, there is no way to "undo" the delivered single bolus dose.

However, in some instances, a user may deliver what is known as an "extended bolus" that is a bolus dosage that is divided into a bolus portion and an extended portion. In more detail, instead of delivering the entire bolus dosage at once, a portion of the bolus dosage, e.g., the "bolus portion" be delivered initially, and the remainder of the bolus dosage, the "extended portion," may be delivered over a period of time, such as 30 minutes, or the like. The "extended portion" may be delivered in amounts greater than a basal dosage delivery but may also be delivered contemporaneously with the basal dosage. In an example, the "bolus portion" may be dosage amount that is approximately 50% of the bolus dosage and the "extended portion" may be a dosage equal to the remaining amount of the dosage portion. The "extended portion" of the extended bolus may be delivered contemporaneously with the basal dosages.

In an extended bolus example, when *CHOₐ* is < 0, and the bolus is an extended bolus that is still being delivered, the AMD system may cancel or decrease any remaining bolus amount of the "extended portion" by an amount of insulin corresponding to the unaccounted for carbohydrate (i.e., unaccounted event information). The decrease to the remaining bolus amount may, for example, be determined by using a negative value for *CHOₐ*(*k*) in Equation 15 above. Such a situation may arise when the user may input a number of expected carbohydrates or a size of a meal prior to being served the meal. The AMD system may begin delivering an extended bolus in preparation for ingestion of the meal. However, upon being served the meal, the user realizes that they overestimated the amount of carbohydrates or the size of the meal. Upon realizing their mistake, the user may revise their carbohydrate or meal size input to the AMD system with an updated carbohydrate amount that is lower than the previous carbohydrate amount or an updated meal size that is smaller than the previously input meal size. In response, the AMD system may take actions to reduce the extended portion of the extended bolus as explained above.

Alternatively, when *CHOₐ* > 0, an additional positive bolus Bₐ accounting for the additional carbohydrate amount *CHOₐ* may be delivered if the additional carbohydrate correction is positive, with an *IOB* adjustment based on the cycle *k_{lastbolus}* at the time of the most recent meal ingestion indication, but no older than 30 minutes prior to the current request. Additionally, when *CHOₐ*(*k*) >0 and additional positive bolus Bₐ of medicament has to be delivered, the entire amount of the additional medicament Bₐ may be delivered in a single "extra" bolus that is in addition to any earlier bolus, or the Bₐ may be delivered in stages, like an extended bolus, where the additional medicament is delivered in addition to any earlier bolus plus any extended amount. In a further alternative, the additional medicament may be delivered as an additional extended bolus, which is a combination of the previous examples, where part of the additional medicament is delivered as a bolus that is in addition to an earlier bolus, and the remaining part of the additional medicament is delivered as an additional extended bolus that is in addition to the extended portion of any earlier determined extended bolus.

In yet another alternative when the additional carbohydrate correction is negative (i.e., *CHOₐ*(*k*) < 0), the insulin onboard (IOB) amount in the current cycle *k* may be increased to account for the potential over-delivery that occurred in the most recent meal bolus request and enable subsequent insulin deliveries to be reduced. In addition, any extended bolus that is being delivered is stopped, or, if the extended bolus is scheduled to be delivered, it is canceled before delivery.

The insulin onboard (IOB) amount in the current cycle *k*, *IOB_{new}*(*k*)*,* may be determined by using, for example, the following: ${IOB}_{new} \left(k\right) = \left\{\begin{matrix}IOB \left(k\right) - \frac{{CHO}_{a} \left(k\right)}{500 / TDI} & {CHO}_{a} \left(k\right) < 0 \\ IOB \left(k\right) & {CHO}_{a} \left(k\right) > 0\end{matrix}\right.$ Equation 16 where *CHOₐ*(*k*) is the adjusted carbohydrate that will be entered as the correct amount of carbohydrate with respect to the previously entered amount of carbohydrate, and that may also have a positive or negative value with respect to the previously entered amount of carbohydrates, and *k* is the current operational cycle.

In an example, the previously entered (and incorrect) amount of carbohydrate may be 60 grams, but the adjusted carbohydrate *CHOₐ*(*k*) may only be 30 grams. In this example, *CHOₐ*(*k*) has a negative value (60 grams minus 30 grams) equaling 30 grams, which is 30 grams less than the previously entered amount of carbohydrate (i.e., - 30 grams). So, the AMD algorithm may be operable to recalculate the amount of insulin onboard by adding the bolus amount of insulin delivered due to the overestimation of ingested carbohydrate that was equal to the amount of previously inputted amount of carbohydrate (e.g., the 60 grams).

The subsequent AMD algorithm requests may then, in part, be affected by the newly calculated IOB values of Equation 16, such as follows: ${U}_{maxIOB} \left(k\right) = \frac{G \left(k\right) - SP \left(k\right)}{1800 / TDI} - {IOB}_{new} \left(k\right)$ Equation 17 in which case, an AMD algorithm may be limited to deliver no more than U_{maxIOB}(k) in the k^{th} cycle.

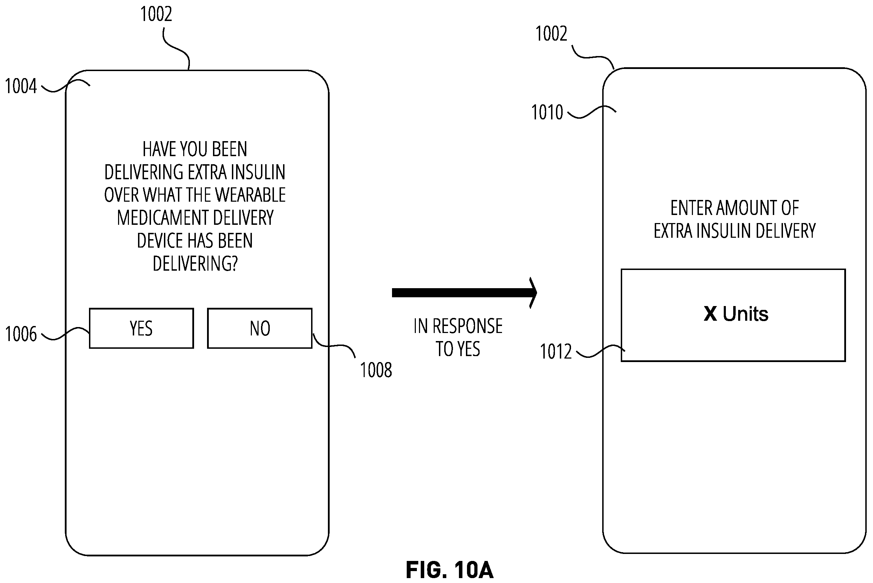

FIG. 10A illustrates an exemplary user interface for updating of exogenous insulin delivery according to the disclosed subject matter.

In this example, the AMD algorithm notes that the user's glucose level is dropping, and it generates a prompt asking the user whether they have informed the AMD system of all the insulin the user is receiving. More broadly, the AMD system is inquiring whether the user has any unaccounted event information that specifically relates to unaccounted for insulin deliveries. For example, this functionality enables the user to update medicament deliveries made separate from a pump or AMD system. In some instances, a user may use a pen injection device or another type of injection device to deliver a dosage of medicament (i.e., an exogenous delivery) outside the use or, and/or the recommendations of the AMD system.

There are a number of ways that the AMD algorithm can determine that a user may be providing themselves with medicament deliveries separate from a pump or AMD system. For example, the exogenous delivery of insulin may be detected by the AMD algorithm by accounting for the frequency of the user's rate of hypoglycemia. Specifically, if the user's rate of hypoglycemia has significantly changed, the user may be prompted based on a value of prompt value *Pₑₓ*, as follows: ${P}_{ex} \left(k\right) = \left\{\begin{matrix}1 & {N}_{hypo , 24 h} \geq 1.5 {N}_{hypo , 24 … 48} \\ 0 & otherwise\end{matrix}\right.$ Equation 18 where 1 means generate a prompt and 0 means do not generate a prompt, and *N* is the number of times the user has been hypoglycemic (e.g., glucose value < 70 mg/dL) and *N*_{*hypo,*24*h*} is the number of times the user was hypoglycemic in the last 24 hours, *N*_{*hypo,*24...48} is the number of times the user was hypoglycemic in the past 24 to 48 hours, and the 1.5 multiplier represents a 50% increase in the number of times the user was hypoglycemic.

In this example, the prompting for exogenous insulin for the current k^{th} cycle, *Pₑₓ*(*k*) may be executed if the number of cycles when the user experienced hypoglycemia (i.e., a glucose value < 70 mg/dL) in the most recent 24 hours may be more than 1.5 times the number of cycles of hypoglycemia between 24 and 48 cycles prior to the current cycle. These are example thresholds only, and additional methods to detect increased rate of hypoglycemia may be incorporated.

Alternatively, whether a user is providing themselves with extra insulin (i.e., insulin beyond what is being delivered as recommended by the AMD algorithm) may be detected by a signature rate (i.e., specific to a user) of downward glucose trajectory that may be characteristic of exogenous insulin delivery. In one example, this may be detected by calculating the residuals between the AMD algorithm's predicted glucose trajectories, and actual glucose readings, and detecting whether a significant negative bias in the actual glucose readings may be introduced, as follows: ${G}_{p} \left(k\right) = {b}_{1} G \left(k-1\right) + {b}_{2} G \left(k-2\right) + ⋯ {b}_{n} G \left(k-n\right) + I \left(k-1\right) + I \left(k-2\right) + ⋯ I \left(k-n\right)$ Equation 19 $R \left(k+m\right) = {\sum }_{q = 0}^{m} G \left(k+q\right) - {G}_{p} \left(k+q\right)$ Equation 20 ${ROC}_{3} \left(k+m\right) = \frac{R \left(k+m\right) - R \left(k+m-2\right)}{3}$ Equation 21 $If \left\{\begin{matrix}{ROC}_{3} \left(k+m\right) \leq {th}_{1} & {P}_{ex} \left(k+m\right) = 1 \\ {ROC}_{3} \left(k+m\right) > {th}_{1} & {P}_{ex} \left(k+m\right) = 0\end{matrix}\right.$ Equation 22 where, the Rate of Change (*ROC*) of the prediction residuals (i.e., *R*(*k+m*) *- R*(*k+m-*2)) for the previous *k*^{th} to the *k+m*^{th} cycles may be calculated over a fixed set of operational cycles, such as 3 cycles in Equation 22. In Equation 22, the number of operational cycles, 3, is a tunable value. The variable q in the summation is an initial operational cycle of the set of operational cycles in the summation, and *m* is the final operational cycle of the set of operational cycles in the summation.

In Equation 22, if the residual value *ROC₃*(*k + m*) is lower than a certain threshold (i.e., *th₁*), such as a persistent overall negative bias in residuals of the threshold *th₁*, such as -100 mg/dL, a potential exogenous insulin delivery event may be detected, in which case, the prompt value *Pₑₓ*(*k+m*) is equal to 1, which means the process determines a prompt, such as in FIG. 10A or the like, is generated. Otherwise, if the residual value *ROC₃*(*k + m*) is greater than the threshold *th₁*, the prompt value *Pₑₓ(k+m)* is equal to 0, which means the process determines a prompt is not generated.

When a prompt is presented (i.e., *Pₑₓ*(*k+m*) is equal to 1), upon receipt of the unaccounted event information of the extraneous medicament deliveries (e.g., extra amounts of insulin being delivered), the AMD algorithm may modify medicament delivery parameters, such as total daily insulin delivery (TDI) estimates and insulin onboard (IOB) constraints, so as to optimize the AMD algorithm behavior for the user.

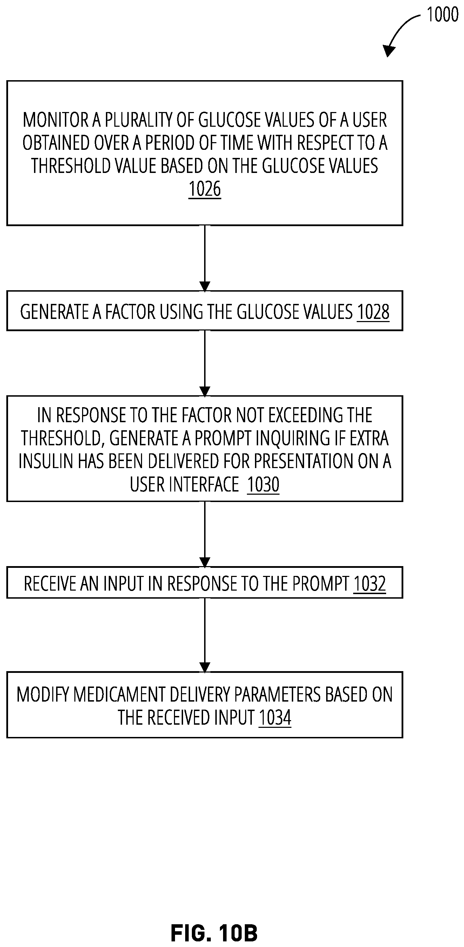

FIG. 10B illustrates an exemplary process for updating of the exogenous insulin delivery according to the disclosed subject matter by utilizing the above Equations 19-22. Although the example process 1000 depicts a particular sequence of operations, the sequence may be altered without departing from the scope of the present disclosure. For example, some of the operations depicted may be performed in parallel or in a different sequence that does not materially affect the function of the process 1000. In other examples, different components of an example device or system that implements the process 1000 may perform functions at substantially the same time or in a specific sequence.

According to some examples, a processor executing an AMD algorithm may implement the process 1000, by monitoring a number of glucose values of a user obtained over a period of time, in particular with respect to a threshold value based on the glucose values at step 1026. The threshold value may be a value indicative of a change in glucose generated based on the glucose values.

At step 1028, the processor may be operable to generate a factor using the glucose values, in particular to generate a factor characterizing a trend in the glucose values over the period of time. In the context of the present disclosure, the "factor" is a quantitative value or parameter derived from physiological data (including, for example, glucose measurements and insulin delivery) and serves as the basis for determining whether to trigger a prompt to the user regarding possible unaccounted insulin delivery events. The factor may take various forms, including counts, rates, ratios, residual values, or trends, as further described below. For example, the processor, when generating the factor may be operable to count a number of respective glucose values from an earlier portion of the period of time that were below a hypoglycemic threshold, in particular wherein the hypoglycemic threshold is a glucose concentration value, and count a number of respective glucose values from a later portion of the period of time that were below the hypoglycemic threshold. In the example, the earlier portion and the later portion are distinct from one another (i.e., they do not overlap). Based on the number of respective glucose values from the later portion being greater than the number of respective glucose values from the earlier portion by a set amount, in particular wherein the set amount is defined by a multiplier applied to the number of respective glucose values from the earlier portion, the processer may proceed with generating the factor.

In a more specific example, the earlier portion of the period of time may be a window of 24 to 48 hours prior to the current cycle, and the later portion of the period of time may be a window of the most recent 24 hours prior to the current cycle. The hypoglycemic threshold, being a glucose concentration value, may be, for example, 70 mg/dL. The set amount by which the number of respective glucose values from the later portion exceeds the number of respective glucose values from the earlier portion may be defined by a multiplier, such as 1.5 times (i.e., a 50% increase), applied to the number of respective glucose values from the earlier portion. Other multipliers, time windows, and hypoglycemic thresholds may be used depending on the user's physiological characteristics and treatment goals.

In other embodiments, the factor may be a function of the number or rate of hypoglycemic events in defined time periods, a ratio comparing such rates across periods (for example, a ratio of *N*_{*hypo,*24*h*}/*N*_{*hypo,*24...48}, see equation 18 above), or any other event-based or statistical value constructed from sensor data. The factor may also include, but is not limited to, values based on prediction modeling, as detailed in the next paragraph. In some embodiments, the threshold may be a fixed or preset value, while in other embodiments, the threshold may be determined dynamically based on the plurality of glucose values obtained over time, such as by computing a statistical property (mean, median, percentile, or moving average) of the monitored values, counted values above or below a threshold, or residuals.

In a more detailed example, the processor, when generating the factor, may be operable to generate estimates of glucose values based on respective glucose values of the plurality of glucose values of the user obtained over the period of time and amounts of insulin delivered that coincide with respective glucose values of the plurality of glucose values. The processor may be operable to determine a residual value between the estimates of glucose values and the respective glucose values. The processor may be operable to generate a factor based on the value of the residual values as compared to a threshold value. In particular, Accordingly, the factor can encompass either event/rate-based metrics (for example as per Equation 18) or residual/predictive modeling-based metrics (for example as per Equations 20 and 21), or any combination thereof, for detecting changes in glycemic pattern that may indicate unaccounted medicament delivery. In particular, the processor may be operable to determine a plurality of residual values, each residual value being a difference between a respective estimate of the glucose values and a corresponding respective glucose value of the plurality of glucose values. The processor may be operable to generate the factor as a rate of change of the residual values over a set of operational cycles, such as 3 operational cycles, wherein the threshold value is a negative bias threshold indicative of a persistent negative bias in the residual values, such as -2 mg/dL/min. For example the bias threshold may be between about -1 mg/dl/min to about -6 mg/dl/min, in particular between about -2 mg/dL/min to about -4 mg/dL/min. The number of operational cycles and the negative bias threshold are tunable values that may be adjusted based on the user's physiological characteristics and treatment goals.

The value of the factor is used to determine whether a prompt should be presented or not. For example, at step 1030, the processor may generate a prompt inquiring if extra insulin has been delivered for presentation on a user interface in response to the factor not exceeding the threshold. Should the factor exceed the threshold, a prompt is not generated, and the process 1000 may terminate. In one example, the system may generate and assign an indicator variable (e.g., *Pₑₓ* as per Equation 18) as a function of the factor and defined threshold, where the indicator is set to 1 to trigger a prompt and set to 0 otherwise. Thus, the factor serves as the underlying detection value, while the indicator or flag determines prompt presentation.

The processor may be operable, via the user interface showing a user revision screen 1010, to receive an input in response to the prompt at step 1032.

At step 1034, the process 1000 includes modifying medicament delivery parameters based on the received input.

Modification of the TDI estimates and IOB constraints are just two example methods of adjusting AMD algorithm behaviors based on user indication of unaccounted for insulin deliveries, and additional adjustments may also be made to other AMD algorithm behaviors, such as incorporation of new insulin delivery values to the AMD algorithm's predicted glucose trajectories, modification of other constraints such as a limitation of maximum insulin deliveries at any fixed periods, or the like.

FIG. 11 illustrates an exemplary medicament delivery system operable to implement the examples disclosed herein.

The medicament system 1100 is suitable for delivering a liquid medicament, such as insulin to a user in accordance with the disclosed embodiments. The medicament system 1100 may include a wearable medicament delivery device 1102, a controller 1104 and an analyte sensor(s) 1106.

The automated medicament delivery system 1100 may comprise a wearable medicament delivery device 1102, a controller 1104, and an analyte sensor(s) 1106. Additionally, the automated medicament delivery system 1100 may include cloud-based services 1110, a network 1112, and a computing device 1114. Also, the automated medicament delivery system 1100 may enable additional sensors, communication devices, and/or data presentation devices, such as a fitness device 1116, a smart accessory device 1118, and another wearable device 1120, to join the system 1100.

The controller 1104 may include a communication circuitry 1122, a transceiver 1124, a transceiver 1126, a user interface 1128, a processor 1130, a memory 1132, other data 1134, a control/AMD application 1136, and settings 1138.

The wearable medicament delivery device 1102 may include a memory 1142, a processor 1150, a user interface 1152, a pump 1154, a reservoir 1156, and a communication circuitry 1158.

The wearable medicament delivery device 1102, the controller 1104, and analyte sensor(s) 1106 are operable to establish communication links via communication circuitry within each (not shown for the analyte sensor(s) 1106, such as communication link 1140, communication link 1164, communication link 1166, and communication link 1168. The communication links 1140, 1164, 1166, and/or 1168 may be any wired or wireless communication link operated according to any known communications protocol or standard, such as Bluetooth^{®}, NFC, Wi-Fi, a near-field communication standard, a cellular standard, or any other wireless protocol. For example, the analyte sensor(s) 1106 may communicate with the wearable medicament delivery device 1102 via a wireless communication link 1140 and/or may communicate with the controller 1104 via a wireless communication link 1166. The wearable medicament delivery device 1102 and controller 1104 may communicate via communication link 1168, additional communication links may enable communication between the controller 1104, the analyte sensor(s) 1106, the wearable medicament delivery device 1102 and external networks, such as network 1112 and cloud-based services 1110.

The memory 1142 may include settings 1144, a control application 1146, and other data 1148.

The wearable medicament delivery device 1102 may be a wearable device that is worn on the body of the user. The wearable medicament delivery device 1102 may be directly coupled to a user (e.g., directly attached to a body part and/or skin of the user via an adhesive, or the like). In an example, a surface of the wearable medicament delivery device 1102 may include an adhesive to facilitate attachment to the skin of a user.

The wearable medicament delivery device 1102 may include a processor 1150. The processor 1150 may be implemented in hardware, software, or any combination thereof. The processor 1150 may, for example, be a microprocessor, a logic circuit, a field programmable gate array (FPGA), an application specific integrated circuit (ASIC) or a microprocessor coupled to a memory. The processor 1150 may maintain a date and time as well as be operable to perform other functions (e.g., calculations or the like). The processor 1150 may be operable to execute a control AMD application 1146 stored in memory 1142 that enables the processor 1150 to direct operation of the wearable medicament delivery device 1102. The control AMD application 1146 may control insulin delivery to the user by an AMD control approach as described herein. For example, the control/AMD application 1136 may be operable to implement an AMD algorithm. The memory 1142 may hold settings 1144 for a user, such as AMD algorithm settings, such as maximum insulin delivery, insulin sensitivity settings, total daily insulin (TDI) settings and the like. Memory 1142 may also store other data 1148, such as ketone values, total daily insulin values, glucose measurement values from analyte sensor(s) 1106 or controller 1104, insulin dosage amounts (both basal and bolus) and the like from previous minutes, hours, days, weeks, or months.

The analyte sensor(s) 1106 may include a continuous glucose monitor 1160a and/or a ketone sensor 1162a, when the analyte sensor(s) 1106 are not co-located with the wearable medicament delivery device 1102. However, if the analyte sensor(s) 1106 is co-located with the wearable medicament delivery device 1102, a continuous glucose monitor 1160b and/or a ketone sensor 1162b may be incorporated in or about the wearable medicament delivery device 1102.

The analyte sensor(s) 1106 may be operable to collect physiological condition data, such as ketone values, ketone values with a time stamp, glucose measurement values (also referred to herein as "blood glucose values" or "blood glucose"), glucose measurement values and a timestamp, and the like. In some examples, the ketone values and/or the glucose values are shared with the wearable medicament delivery device 1102, the controller 1104, or both. In an additional example, the analyte sensor(s) 1106 may include multiple sensors, such as a continuous glucose monitor 1160a and a ketone sensor(s) 1162a. In a further example, the wearable medicament delivery device 1102 may optionally include a continuous glucose monitor 1160b and a ketone sensor 1162b, which may be removably incorporated or fully integrated within the wearable medicament delivery device 1102. For example, the continuous glucose monitor 1160b and the ketone sensor 1162b may be incorporated in one or more housings of the wearable medicament delivery device 1102.

For example, the communication circuitry 1158 of the wearable medicament delivery device 1102 may be operable to communicate with the analyte sensor(s) 1106 and the controller 1104 as well as the devices 1116, 1118 and 1120. The communication circuitry 1158 may be operable to communicate via Bluetooth, cellular communication, near field communication (NFC) and/or other wireless protocols. While not shown, memory 1142 may include both primary memory and secondary memory. The memory 1142 may include random access memory (RAM), read only memory (ROM), local storage, magnetic storage, removable storage media, solid state storage or the like.

The wearable medicament delivery device 1102 may include a reservoir 1156. The reservoir 1156 may be operable to store liquid drugs, medicaments, or therapeutic agents suitable for automated delivery. For example, medicament in liquid form capable of being administered by a medicament delivery device via a subcutaneous cannula, may include medicaments, such as, insulin, glucagon-like peptide-1 (GLP-1) receptor agonist, pramlintide, glucose-dependent insulinotropic polypeptide (GIP), glucagon, other hormones, or co-formulations of two or more of glucagon, GLP-1, pramlintide, and insulin; as well as pain relief drugs, such as opioids or narcotics (e.g., morphine, or the like), methadone, blood pressure medicines, weight loss medicaments, chemotherapy drugs, fertility drugs, various coformulations of any of the above, or the like.

A fluid path to the user may be provided via tubing and a needle/cannula (not shown). The fluid path may, for example, include tubing coupling the wearable medicament delivery device 1102 to the user (e.g., via tubing coupling a needle or cannula to the reservoir 1156). The tubing may be internal to the wearable medicament delivery device 1102 (i.e., a tubeless wearable medicament delivery device 1102). In other examples, the tubing may extend to an infusion site device, in which case the wearable medicament delivery device 1102 may be referred to as a tubed wearable medicament delivery device 1102. The wearable medicament delivery device 1102 may be operable based on control signals from the processor 1150 to expel the liquid drugs, medicaments, or therapeutic agents, such as insulin, from the reservoir 1156 to deliver doses of the drugs, medicaments, or therapeutic agents, such as the insulin, to the user via the fluid path. The processor 1150 may be operable to cause insulin to be expelled from the reservoir 1156.

The wearable medicament delivery device 1102 may also include a user interface 1152, such as an integrated display device for displaying information to the user, and in some embodiments, receiving information from the user. For example, the user interface 1152 may include a touchscreen and/or one or more input devices, such as buttons, knob(s), or a keyboard that enable a user to provide an input.

In addition, the processor 1150 may be operable to receive data or information from the analyte sensor(s) 1106 as well as other devices that may be operable to communicate with the wearable medicament delivery device 1102.

The wearable medicament delivery device 1102 and/or the controller 1104 may interface with a network 1112. The network 1112 may include a local area network (LAN), a wide area network (WAN) or a combination therein. A computing device 1114 may be interfaced with the network, and the computing device may communicate with the wearable medicament delivery device 1102. The computing device 1114 may be a healthcare provider device through which a user's controller 1104 may interact to obtain information, store settings and the like. The AMD algorithm operating, as or in cooperation with, a control application (not shown), such as the control/AMD application 1136, may present a graphical user interface on the computing device 1114 enabling the input and presentation of information related to the AMD algorithm and the example techniques and processes described herein.

The medicament system 1100 may include an analyte sensor(s) 1106 for sensing the levels of one or more analytes of a user. The analyte levels may be used as physiological condition data and be sent to the controller 1104 and/or the wearable medicament delivery device 1102. The analyte sensor(s) 1106 may be coupled to the user by, for example, adhesive or the like and may provide information or data on one or more medical conditions and/or physical attributes of the user. The analyte sensor(s) 1106 may be a continuous glucose monitor (CGM), ketone sensor, or another type of device or sensor(s) that provides glucose measurements and/or ketone measurements. The analyte sensor(s) 1106 may be physically separate from the wearable medicament delivery device 1102 or may be an integrated component thereof. The analyte sensor(s) 1106 may provide the processor 1130 and/or processor 1150 with physiological condition data indicative of measured or detected glucose levels of the user. The information or data provided by the sensor(s) 1106 may be used to modify a medicament delivery schedule and thereby cause the adjustment of medicament operations of the wearable medicament delivery device 1102.

In the depicted example, the controller 1104 may include a processor 1130 and a memory 1132. The controller 1104 may be a special purpose device, such as a dedicated personal diabetes manager (PDM) device. The controller 1104 may be a programmed general-purpose device that is a portable electronic device, such as any portable electronic device, smartphone, smartwatch, fitness device, tablet or the like including, for example, a dedicated processor, such as processor, a microprocessor, or the like. The controller 1104 may be used to program or adjust operation of the wearable medicament delivery device 1102 and/or the sensor(s) 1106. The processor 1130 may execute processes to control the delivery of the medicament, drug, or therapeutic agent to the user for the purpose of managing a user's blood glucose and/or ketone levels. The processor 1130 may also be operable to execute programming code stored in the memory 1132. For example, the memory 1132 may be operable to store a control application 1136, such as an AMD algorithm for execution by the processor 1130. The control application 1136 may be responsible for controlling the wearable medicament delivery device 1102, including the automated delivery of medicament, a drug, or therapeutic agent based on recommendations and instructions from the AMD algorithm, such as those recommendations and instructions described herein.

The memory 1132 may store one or more applications, such as control application 1136, and settings 1138 for the medicament device 1102 like those described above. In addition, the memory 1132 may be operable to store other data 1134 and/or computer programs, such as medicament history, glucose measurement values over a period of time, total daily insulin values, ketone values, and the like. For example, the memory 1132 is coupled to the processor 1130 and operable to store programming instructions, such as the control AMD application 1136 and settings 1138, and data, such as other data 1134, related to a glucose of a user and/or data related to an amount of insulin expelled by the wearable medicament delivery device 1102.

The controller 1104 may include a user interface 1128 (UI) for communicating with the user. The user interface 1128 may include a display, such as a touchscreen, for displaying information. The touchscreen may also be used to receive input when it is a touch screen. The user interface 1128 may also include input elements, such as a keyboard, button, knob, or the like. In an operational example, the user interface 1128 may include a touchscreen display (including a display and user input circuitry, such as touch sensitive circuits and the like) controllable by the processor 1130 and be operable to present a graphical user interface and receive inputs via the user input circuitry, the touchscreen display may be operable to generate a signal indicative of the respective diet types, number of days, diet cycles, keto days, sick day mode, sickness symptoms, intermittent fasting mode, fasting mode, religious fasting mode, location information, calendar information, or the like. The touchscreen display, under control of the processor 1130, may be operable to, in response to the received input, generate a response to a user's drug treatment regimen as well as cause the presentation of prompts on a graphical user interface as described with reference to the later examples described with reference to FIGs. 1-10B. The graphical user interfaces discussed herein with respect to the later examples may be generated by the processor 1130 of the controller 1104 and be presented on the UI 1128.

The controller 1104 may interface via a wireless communication link of the wireless communication links 1164 with a network, such as a LAN or WAN or combination of such networks that provides one or more servers or cloud-based services 1110 via communication circuitry 1122. The communication circuitry 1122, which may include transceivers 1124 and 1126, may be coupled to the processor 1130. The communication circuitry 1122 may be operable to transmit communication signals (e.g., command and control signals) to and receive communication signals (e.g., via transceivers 1126 or 1124) from the wearable medicament delivery device 1102 and the analyte sensor(s) 1106. In an example, the communication circuitry 1122 may include a first transceiver, such as 1124, that may be a Bluetooth transceiver, which is operable to communicate with the communication circuitry 1122 of the wearable medicament delivery device 1102, and a second transceiver, such as 1126, that may be a cellular or Wi-Fi transceiver operable to communicate via the network 1112 with computing device 1114 or with cloud-based services 1110.

The cloud-based services 1110 may be operable to receive and store user history information, such as glucose measurement values over a set period of time (e.g., days, months, years), a medicament history that includes insulin delivery amounts (both basal and bolus dosages) and insulin delivery times, types of insulin delivered, indicated meal times, glucose measurement value trends or excursions or other user-related diabetes treatment information, specific factor settings including default settings, present settings and past settings, or the like.

Other devices, like smart accessory device 1118 (e.g., a smartwatch or the like), fitness device 1116 and other wearable device 1120 may be part of the medicament system 1100. These devices may communicate with the wearable medicament delivery device 1102 to receive information and/or issue commands to the wearable medicament delivery device 1102. These devices 1116, 1118 and 1120 may execute computer programming instructions to perform some the control functions otherwise performed by processor 1150 or processor 1130. These devices 1116, 1118 and 1120 may include user interfaces, such as touchscreen displays for displaying information such as current glucose level, medicament on board such as insulin on board, medicament history, or other parameters or treatment-related information and/or receiving inputs, such as those described with reference to the examples of FIGs. 1-3. The display may, for example, be operable to present a graphical user interface for providing input, such as a request to change the basal insulin dosage or delivery of a bolus of insulin. These devices 1116, 1118 and 1120 may also have wireless communication connections with the sensor(s) 1106 to directly receive glucose level data or receive in parallel a presentation of the graphical user interface as shown in FIG. 11.

In another operational example, the controller 1104 may be operable to execute programming code that causes the processor 1130 of the controller 1104 to perform the following functions. The processor 1130 of the controller 1104 may execute an AMD algorithm that is one of the control applications 1136 stored in the memory 1132. The processor may be operable to present graphical user interfaces as described herein on a user interface that is at least one component of the user interface 1128. The user interface 1128 may be a touchscreen display controlled by the processor 1130, and the user interface 1128 is operable to present a graphical user interface that offers an input of a subjective insulin need parameter usable by the AMD algorithm. The processor 1130 may cause presentation on a user interface of a user device, a graphical user interface offering an input device that enables input of a subjective medicament need parameter (e.g., subjective to the user, or based on the user's desire). The AMD algorithm may generate instructions for the pump 1154 to deliver basal medicament to the user or the like.

The processor 1130 is also operable to collect physiological condition data related to the user from sensors, such as the analyte sensor(s) 1106 or heart rate data, for example, from the fitness device 1116 or the smart accessory device 1118. In an example, the processor 1130 executing the AMD algorithm may determine a dosage of medicament to be delivered based on the collected physiological condition of the user and/or a specific factor determined based on the subjective medicament need parameter. The processor 1130 may output a control signal via one of the transceivers 1124 or 1126 to the wearable medicament delivery device 1102. The outputted signal may cause the processor 1150 to deliver command signals to the pump 1154 to deliver an amount of the determined dosage of medicament in the reservoir 1156 to the user based on an output of the AMD algorithm. The processor 1130 may also be operable to perform calculations to update or establish settings of the AMD algorithm as discussed as herein. Modifications to the AMD algorithm settings may be stored in the memory 1132, for example, as settings 1144.

A wearable medicament delivery device 1102, typically, has a lifecycle that is based on the amount of liquid drug that is stored in a reservoir 1156 of the wearable medicament delivery device 1102 and/or the amount of the liquid drug delivered to the user. An AMD application or algorithm may use a number of parameters, such as glucose measurement values, total daily medicament, medicament onboard, and the like, when making the determination of an amount of the liquid drug to have delivered.

While the system 1100 is described with reference to delivery of insulin and the use of an AMD algorithm, the system 1100 may be operable to implement a medicament regimen via a medication delivery algorithm using a number of different liquid or therapeutic drugs and medicaments as described above.

Software related implementations of the techniques described herein, such as the examples described with reference to FIGs. 1-12 may include, but are not limited to, firmware, application specific software, applet, or any other type of computer readable instructions that may be executed by one or more processors. The computer readable instructions may be provided via non-transitory computer-readable media. The various elements of the processes described with reference to the figures may be implemented in devices, apparatuses or systems that may include various hardware elements, software elements, or a combination of both. Examples of hardware elements that may be the basis for a computer processor may include structural members, logic devices, components, processors, microprocessors, circuits, processors, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), memory units, logic gates, registers, semiconductor device, chips, microchips, chip sets, and so forth. Examples of software elements may include software components, programs, applications, computer programs, application programs, system programs, software development programs, machine programs, operating system software, middleware, firmware, software modules, routines, subroutines, functions, processes, procedures, software interfaces, application program interfaces (API), instruction sets, computing code, computer code, code segments, computer code segments, words, values, symbols, or any combination thereof.

Some examples of the disclosed device or processes may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined solely by the preceding illustrative description.

Program aspects of the technology may be thought of as "products" or "articles of manufacture" typically in the form of executable code and/or associated data that is carried on or embodied in a type of non-transitory, machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, novel subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible considering this disclosure. It is intended that the scope of the present disclosure be limited not by this detailed description, but rather by the claims appended hereto. Future filed applications claiming priority to this application may claim the disclosed subject matter in a different manner and may generally include any set of one or more features as variously disclosed or otherwise demonstrated herein.

Although the present invention is defined in the attached claims, it should be understood that the present invention can also (alternatively) be defined in accordance with the following embodiments:
1. A medicament delivery system, comprising:
   a wearable medicament delivery device operable to communicate with an external device and to expel medicament from a reservoir;
   a data storage to maintain treatment program parameters during an adaptation cycle of the wearable medicament delivery device;
   a touchscreen display; and
   a processor coupled to the touchscreen display and operable to communicate with the wearable medicament delivery device and execute a control application, the processor operable to:
      evaluate the treatment program parameters for the adaptation cycle;
      receive, via a user interface presented on the touchscreen display, additional unaccounted event information regarding an unaccounted event during the adaptation cycle;
      revise treatment program parameters based on the additional unaccounted event information; and
      estimate medicament dosages to be expelled from the wearable medicament delivery device.
2. The medicament delivery system of embodiment 1, wherein the unaccounted event includes at least one of an extraneous medicament delivery, or omitted physical activity.
3. The medicament delivery system of c embodiment 2, wherein the extraneous medicament delivery is an amount of medicament delivered via a device other than the wearable medicament delivery device.
4. The medicament delivery system of embodiment 2, wherein the omitted physical activity is at least one of: exercise, or unplanned physical exertion.
5. The medicament delivery system of embodiment 1, wherein the adaptation cycle is a lifecycle of the wearable medicament delivery device.
6. The medicament delivery system of embodiment 1, wherein the adaptation cycle is a process executed by an automated medicament delivery algorithm when the wearable medicament delivery device is activated for use.
7. The medicament delivery system of embodiment 1, wherein the treatment program parameters include total daily dosage and target glucose measurement values or ranges.
8. A medicament delivery system, comprising:
   a wearable medicament delivery device operable to communicate with an external device and to expel medicament from a reservoir;
   a data storage maintaining a user history related to the wearable medicament delivery device; and
   a processor coupled to the data storage and operable to communicate with the wearable medicament delivery device and execute a control application, the processor operable to:
      present in a user interface prompts for a user to enter unaccounted event information for an event that occurred at least a set time period prior to presentation of the prompt;
      receive the unaccounted event information;
      evaluate the received unaccounted event information; and
      based on a result of the evaluation, determine whether to adjust one or more of: a bolus amount, an amount of insulin on board, or a maximum amount of insulin to deliver in a next operational cycle.
9. The medicament delivery system of embodiment 8, wherein the unaccounted event information includes past meal information for a meal that occurred at least a set time period prior to presentation of the prompt.
10. The medicament delivery system of embodiment 8, wherein the set time period is at least 30 minutes prior to the presentation of the prompt.
11. The medicament delivery system of embodiment 8, wherein the set time period is at least 90 minutes prior to the presentation of the prompt.
12. The medicament delivery system of embodiment 8, wherein the processor is further operable to, based on the received unaccounted event information indicating an unrecorded meal, re-classify insulin delivered by the wearable medicament delivery device beyond a basal amount during a period of time after the unrecorded meal as meal insulin requirements rather than glucose excursion correction insulin requirements.
13. A medicament delivery system, comprising:
   a wearable medicament delivery device operable to communicate with an external device and to expel medicament from a reservoir;
   a data storage maintaining treatment program parameters during an adaptation cycle of the wearable medicament delivery device;
   a touchscreen display; and
   a processor coupled to the touchscreen display and operable to communicate with the wearable medicament delivery device and execute a control application, the processor operable to:
      after start of an extended bolus to compensate for a meal, present a user interface on the touchscreen display enabling entry of a revised carbohydrate amount for the meal to replace a previous estimate;
      in response to the entry of the revised carbohydrate amount for the meal, determine a remainder of the started extended bolus yet to be delivered;
      calculate an updated dosage for the started extended bolus;
      revise the remainder of the started extended bolus yet to be delivered based on the calculated updated dosage for the started extended bolus; and
      cause the revised remainder to be expelled by the wearable medicament delivery device.
14. The medicament delivery system of embodiment 13, wherein the processor is further operable to, in response to the revised carbohydrate amount being less than the previous estimate, decrease the remainder of the started extended bolus yet to be delivered by an amount of insulin corresponding to a difference between the previous estimate and the revised carbohydrate amount.
15. A medicament delivery system, comprising:
   a processor; and
   a memory storing instructions that, when executed by the processor, the processor is operable to:
      enter a first amount of carbohydrate for a meal into a bolus calculator, wherein the first amount of carbohydrate is an initial estimate of an amount of carbohydrate in the meal;
      generate a bolus dosage based on the entered first amount of carbohydrate;
      enter a second amount of carbohydrate for the meal into the bolus calculator,
   wherein the second amount of carbohydrate is an updated estimate of the first amount of carbohydrate in the meal;
      evaluate the first amount of carbohydrate and the second amount of carbohydrate; and
      generate an adjusted bolus dosage based on the entered first amount of carbohydrate, the entered second amount of carbohydrate, and the generated bolus dosage.
16. The medicament delivery system of embodiment 15, wherein the processor is operable to:
   cause delivery of the adjusted bolus dosage by expelling an amount of medicament of the adjusted bolus dosage.
17. The medicament delivery system of embodiment 15, wherein the processor, when evaluating the first amount of carbohydrate and the second amount of carbohydrate, is further operable to determine a remainder of an extended bolus yet to be delivered based on a duration defined by a number of minutes since the meal was ingested, and account for the remainder when generating the adjusted bolus dosage.
18. A medicament delivery system, comprising:
   a processor; and
   a memory storing instructions that, when executed by the processor, the processor is operable to:
      monitor a plurality of glucose values of a user obtained over a period of time with respect to a threshold value, wherein the threshold value is a value generated based on the glucose values;
      generate a factor using the glucose values;
      determine whether the factor exceeds the threshold value;
      generate a prompt inquiring if extra insulin has been delivered for presentation on a user interface in response to the factor not exceeding the threshold value, wherein a prompt is not generated when the factor does exceed the threshold value;
      receive an input in response to the prompt; and
      modify medicament delivery parameters based on the received input.
19. The medicament delivery system of embodiment 18, wherein the processor, when generating the factor, is further operable to:
   count a number of respective glucose values from an earlier portion of the period of time that were below a hypoglycemic threshold;
   count a number of respective glucose values from a later portion of the period of time that were below a hypoglycemic threshold, wherein the earlier portion and the later portion are distinct from one another; and
   based on the number of respective glucose values from the later portion being greater than the number of respective glucose values from the earlier portion by a set amount, proceed with generating the factor.
20. The medicament delivery system of embodiment 18, wherein the processor is further operable to:
   generate estimates of glucose values based on respective glucose values of the plurality of glucose values of the user obtained over the period of time and amounts of insulin delivered that coincide with respective glucose values of the plurality of glucose values; and
   determine a residual value between the estimates of glucose values and the respective glucose values.

## Claims

1. A medicament delivery system, comprising:
a wearable medicament delivery device operable to communicate with an external device and to expel medicament from a reservoir;
a data storage to maintain treatment program parameters during an adaptation cycle of the wearable medicament delivery device;
a display; and
a processor coupled to the display and operable to communicate with the wearable medicament delivery device and execute a control application, the processor operable to:
evaluate the treatment program parameters for the adaptation cycle;
receive, via a user interface presented on the display, additional unaccounted event information regarding an unaccounted event during the adaptation cycle;
revise treatment program parameters based on the additional unaccounted event information; and
estimate medicament dosages to be expelled from the wearable medicament delivery device based on the revised treatment program parameters.

2. The medicament delivery system of claim 1, wherein the unaccounted event includes at least one of an extraneous medicament delivery, or omitted physical activity, in particular wherein the extraneous medicament delivery is an amount of medicament delivered via a device other than the wearable medicament delivery device and/or wherein the omitted physical activity is at least one of: exercise, or unplanned physical exertion.

3. The medicament delivery system of any one of claims 1 to 2, wherein the adaptation cycle is a lifecycle of the wearable medicament delivery device, or wherein the adaptation cycle is a process executed by an automated medicament delivery algorithm when the wearable medicament delivery device is activated for use.

4. The medicament delivery system of any one of claims 1 to 3, wherein the treatment program parameters include total daily dosage and target glucose measurement values or ranges.

5. A medicament delivery system, comprising:
a wearable medicament delivery device operable to communicate with an external device and to expel medicament from a reservoir;
a data storage maintaining a user history related to the wearable medicament delivery device; and
a processor coupled to the data storage and operable to communicate with the wearable medicament delivery device and execute a control application, the processor operable to:
present in a user interface prompts for a user to enter unaccounted event information for an event that occurred at least a set time period prior to presentation of the prompt;
receive the unaccounted event information;
evaluate the received unaccounted event information; and
based on a result of the evaluation, determine whether to adjust one or more of: a bolus amount, an amount of insulin on board, or a maximum amount of insulin to deliver in a next operational cycle.

6. The medicament delivery system of claim 5, wherein the unaccounted event information includes past meal information for a meal that occurred at least a set time period prior to presentation of the prompt, more specifically wherein the set time period is at least 30 minutes prior to the presentation of the prompt, in particular wherein the set time period is at least 90 minutes prior to the presentation of the prompt.

7. The medicament delivery system of any one of claims 5 or 6, wherein the processor is further operable to, based on the received unaccounted event information indicating an unrecorded meal, re-classify insulin delivered by the wearable medicament delivery device beyond a basal amount during a period of time after the unrecorded meal as meal insulin requirements rather than glucose excursion correction insulin requirements.

8. A medicament delivery system, comprising:
a wearable medicament delivery device operable to communicate with an external device and to expel medicament from a reservoir;
a data storage maintaining treatment program parameters during an adaptation cycle of the wearable medicament delivery device;
a display; and
a processor coupled to the display and operable to communicate with the wearable medicament delivery device and execute a control application, the processor operable to:
after start of an extended bolus to compensate for a meal, present a user interface on the display enabling entry of a revised carbohydrate amount for the meal to replace a previous estimate;
in response to the entry of the revised carbohydrate amount for the meal, determine a remainder of the started extended bolus yet to be delivered;
calculate an updated dosage for the started extended bolus based on the revised carbohydrate amount for the meal;
revise the remainder of the started extended bolus yet to be delivered based on the calculated updated dosage for the started extended bolus; and
cause the revised remainder to be expelled by the wearable medicament delivery device.

9. The medicament delivery system of claim 8, wherein the processor is further operable to, in response to the revised carbohydrate amount being less than the previous estimate, decrease the remainder of the started extended bolus yet to be delivered by an amount of insulin corresponding to a difference between the previous estimate and the revised carbohydrate amount.

10. A medicament delivery system, comprising:
a processor; and
a memory storing instructions that, when executed by the processor, the processor is operable to:
enter a first amount of carbohydrate for a meal into a bolus calculator, wherein the first amount of carbohydrate is an initial estimate of an amount of carbohydrate in the meal;
generate a bolus dosage based on the entered first amount of carbohydrate;
enter a second amount of carbohydrate for the meal into the bolus calculator,
wherein the second amount of carbohydrate is an updated estimate of the first amount of carbohydrate in the meal;
evaluate the first amount of carbohydrate and the second amount of carbohydrate; and
generate an adjusted bolus dosage based on the entered first amount of carbohydrate, the entered second amount of carbohydrate, and the generated bolus dosage.

11. The medicament delivery system of claim 10, wherein the processor is operable to:
cause delivery of the adjusted bolus dosage by expelling an amount of medicament of the adjusted bolus dosage, in particular wherein the processor, when evaluating the first amount of carbohydrate and the second amount of carbohydrate, is further operable to determine a remainder of an extended bolus yet to be delivered based on a duration defined by a number of minutes since the meal was ingested, and account for the remainder when generating the adjusted bolus dosage.

12. A medicament delivery system, comprising:
a processor; and
a memory storing instructions that, when executed by the processor, the processor is operable to:
monitor a plurality of glucose values of a user obtained over a period of time;
generate a factor using the glucose values;
determine whether the factor exceeds a threshold value;
generate a prompt inquiring if extra insulin has been delivered for presentation on a user interface in response to the factor not exceeding the threshold value, wherein a prompt is not generated when the factor does exceed the threshold value;
receive an input in response to the prompt; and
modify medicament delivery parameters based on the received input.

13. The medicament delivery system of claim 12, wherein the processor, when generating the factor, is further operable to:
count a number of respective glucose values from an earlier portion of the period of time that were below a hypoglycemic threshold, in particular wherein the hypoglycemic threshold is a glucose concentration value;
count a number of respective glucose values from a later portion of the period of time that were below a hypoglycemic threshold, wherein the earlier portion and the later portion are distinct from one another; and
based on the number of respective glucose values from the later portion being greater than the number of respective glucose values from the earlier portion by a set amount, proceed with generating the factor, in particular wherein the set amount is defined by a multiplier applied to the number of respective glucose values from the earlier portion.

14. The medicament delivery system of any one of claims 12 or 13, wherein the processor is further operable to:
generate estimates of glucose values based on respective glucose values of the plurality of glucose values of the user obtained over the period of time and amounts of insulin delivered that coincide with respective glucose values of the plurality of glucose values; and
determine a residual value between the estimates of glucose values and the respective glucose values, and
generate a factor based on the value of the residual values as compared to a threshold value, in particular generate a factor as a rate of change of the residual values over a set of operational cycles, wherein the threshold value is a negative bias threshold indicative of a persistent negative bias in the residual values.

15. The medicament delivery system of any one of claims 12 to 14, wherein the threshold value is a preset value or is determined dynamically based on the plurality of glucose values, in particular wherein the threshold value is determined dynamically based on computing a statistical property of the glucose values, counted values above or below a threshold, or residual values.

16. The medicament delivery system of any one of claims 12 to 15, wherein the medicament delivery parameters modified in response to the received input comprise one or more of: a recorded insulin delivery history, an insulin-on-board estimate, a total daily insulin estimate, or a parameter of an automated medicament delivery algorithm.
